(19) Europäisches Patentamt
European Patent Office
Office européen des brevets

(11) Publication number: **0 019 450**
**B1**

(12) # EUROPEAN PATENT SPECIFICATION

(45) Date of publication of patent specification: **23.02.83**

(21) Application number: **80301558.5**

(22) Date of filing: **13.05.80**

(51) Int. Cl.[3]: **C 07 D 239/46,**
C 07 D 239/42,
C 07 D 239/52,
C 07 D 239/70,
C 07 D 239/84,
A 01 N 43/54

(54) **Pyrimidine derivatives and their production and agricultural uses.**

(30) Priority: **15.05.79 JP 60069/79**

(43) Date of publication of application:
**26.11.80 Bulletin 80/24**

(45) Publication of the grant of the patent:
**23.02.83 Bulletin 83/8**

(84) Designated Contracting States:
**CH DE FR GB IT LI**

(56) References cited:
**GB - A - 1 223 686**
**US - A - 3 850 915**

(73) Proprietor: **Takeda Chemical Industries, Ltd.**
**27, Doshomachi 2-chome**
**Higashi-ku Osaka, 541 (JP)**

(72) Inventor: **Konishi, Kazuo**
**9-8, Higashikanmaki 3-Chome**
**Takatsuki Osaka 569 (JP)**
Inventor: **Matsuura, Kazuho**
**8-59, Yamabata-Itchodacho**
**Sakyo-Ku Kyoto 606 (JP)**

(74) Representative: **Laredo, Jack Joseph et al,**
**Elkington and Fife High Holborn House 52/54**
**High Holborn**
**London, WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 019 450 B1

Pyrimidine derivatives and their production and agricultural uses

The present invention relates to novel pyrimidine derivatives, methods for the production thereof, and antimicrobial agents for agricultural uses featured by containing one or more kinds of the derivatives as the active ingredient or ingredients.

More particularly, the invention relates to a pyrimidine derivative of the formula (I):

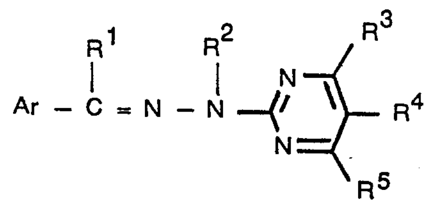

(I),

or a salt thereof,

wherein Ar is phenyl or naphthyl, which may be substituted by $C_{1-4}$ alkyl, $C_{1-4}$ alkoxy, $C_{1-4}$ alkylthio, $C_{1-4}$ alkylsulfinyl, $C_{1-4}$ alkylsulfonyl, halogen, nitro, trifluoromethyl or di-$C_{1-4}$ alkylamino; $R^1$ is $C_{1-4}$ alkoxycarbonyl, phenyl or benzyl, and the phenyl may be substituted by halogen; $R^2$ is hydrogen or $C_{1-4}$ alkyl; $R^3$, $R^4$ and $R^5$ are hydrogen, $C_{1-4}$ alkyl, $C_{2-4}$ alkenyl or $C_{1-4}$ alkoxy, or $R^3$ and $R^4$ or $R^5$ combine with each other to represent trimethylene, tetramethylene or butadienylene;

a method of producing a pyrimidine derivative (I), or a salt thereof, which comprises reacting an aromatic ketone of the formula (II):

$$ArCOR^1 \qquad (II),$$

wherein the symbols in the formula are as defined above,

with 2-pyrimidylhydrazine of the formula (III):

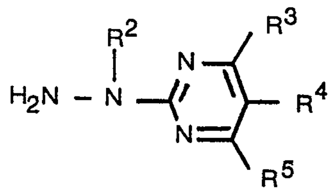

(III),

or a salt thereof,

wherein the symbols in the formula are as defined above;

a method of producing a pyrimidine derivative of the formula (VI):

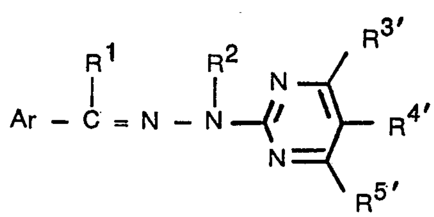

(VI),

or a salt thereof,

wherein the symbols in the formula are as defined below,

which comprises reacting an amidinohydrazone of the formula (IV):

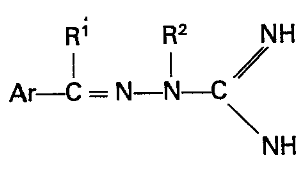

(IV),

or a salt thereof,

wherein the symbols in the formula are as defined above,

with a $\beta$-diketone of the formula (V):

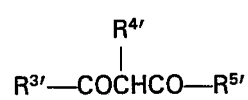

(V)

wherein, in the above formulae IV to VI, $R^{3'}$, $R^{4'}$ and $R^{5'}$ are hydrogen, $C_{1-4}$ alkyl or $C_{2-4}$ alkenyl or

$R^{4'}$ is $C_{1-4}$ alkoxy, or $R^{3'}$ and $R^{4'}$ or $R^{4'}$ and $R^{5'}$ combine with each other to represent trimethylene or tetramethylene; and

an antimicrobial agent for agricultural uses which contains as the active ingredient or ingredients one or more of the pyrimidine derivatives (I) and/or their salts.

The need to increase food production has been, and is at present a most urgent goal. Large numbers of organometallic bactericides and fungicides, mainly organomercurial preparations, have been manufactured in enormous quantities, and have been widely used for many years because of their outstanding bactericidal and fungicidal effects. Apart from the intended effects produced, the use of such antimicrobial agents has brought about simultaneously a variety of undesirable, negative impacts on man and animals and the environment, and has come to present a social problem. Instead of these organometallic-compounds, there have emerged, up to now, antibiotics, organic phosphorus compounds, organic chlorine compounds, and so on. However, in spite of the fact that multiple diseases usually simultaneously affect the cultivation of crops, the currently prevalent, non-metallic antimicrobial agents, as described hereinbefore, all show a strong selectivity and a narrow antimicrobial spectrum, so that many of them are effective merely against a particular, single disease and its effects. Consequently, the use of a mixed preparation by way of a combined utilization of a multiple number of active substances is largely relied upon, inevitably, from the standpoint of labour-saving, simultaneous pest control. This does not necessarily seem desirable in respect of impact on the environment, efficient use of resources, reduction of expenditures, and so on. Furthermore, there still remain some kinds of diseases and their effects which have not been overcome satisfactorily by these antimicrobial agents alternatively introduced. As a disease affecting paddy rice culture, for example, there may be mentioned rice plant blast, sheath blight leaf spot, stem-rot, Helminthosporium leaf spot, and bacterial leaf blight. As regards the first two diseases, there are currently some antimicrobial agents available for individually controlling these to a limited extent, whereas the others have been left unsolved. Especially, the third and fourth diseases have begun to occur conspicuously to the severest degree in recent years, and there is a strong demand an antimicrobial agent with high effectiveness against the two first mentioned diseases, and which simultaneously exhibits a similarly effective control against the others. The same is true for diseases affecting dry field farming, fruit culture, floriculture, and so on, for example, dawny mildew of cucumber, gray mould of strawberry, stem rot of kidney bean, powdery mildew of barley, gray mould of lettuce, late blight of tomato, leaf blight of cucumber, and so on.

As a result of our research in this field, we have unexpectedly discovered that a novel pyrimidine derivative of the formula (I) or a salt thereof, which are somewhat different from the type of compounds used as conventional antimicrobial agents and which have the hydrazone linkage in the molecule, are capable of providing a clear solution to these problems. The present invention is based on this novel finding.

Japanese (Published but not examined) Patent Application No. 12786/1978 describes an antimicrobial agent for agricultural uses, which contains as an active ingredient a pyrimidine derivative of the general formula:

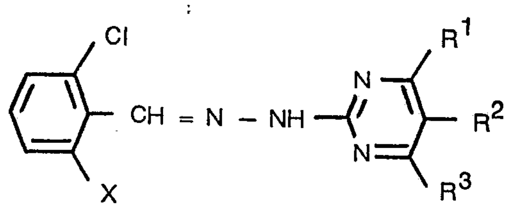

wherein X is Cl or F, and $R^1$, $R^2$ and $R^3$ are hydrogen or a lower alkyl group, at least one of them being a lower alkyl group.

Representative pyrimidine derivatives of the formula just given are as follows:

1. 2-(2,6-dichlorobenzilidenehydrazino)-4-methylpyrimidine.
2. 2-(2,6-dichlorobenzilidenehydrazino)-4,6,-dimethylpyrimidine.
3. 2-(2,6-dichlorobenzilidenehydrazino)-4,5,6-trimethylpyrimidine.
4. 2-(2,6-dichlorobenzilidenehydrazino)-4-ethyl-6-methylpyrimidine.
5. 2-(2,6-dichlorobenzilidenehydrazino)-5-ethyl-4,6-dimethylpyrimidine.
6. 2-(2,6-dichlorobenzilidenehydrazino)-4,6-dimethyl-5-propylpyrimidine.
7. 2-(2-chloro-6-fluorobenzilidenehydrazino)-4,6-dimethylpyrimidine.

The following indicates the results of the antimicrobial activity of the compounds Nos. 1 to 7.

| test microorganism \ representative compound No. | 1 | 2 | 3 | 4 | 5 | 6 | 7 |
|---|---|---|---|---|---|---|---|
| *Pyricularia oryzae* | 12.5 | 1.56 | 0.39 | 6.25 | 0.39 | 0.78 | 3.12 |

A number shows minimum inhibitory concentration (mcg/ml).

(See also Chemical Abstracts, Vol. 89, 1978, page 642, 89: 43476Q).

The second mentioned compound was chosen by us as a control reference compound in the tests discussed below.

As these show, the examples of the present invention within the above noted formula (I) which were compared with this reference compound (referred to as compound (VII) herein) did not show significant differences from the control in respect of the minimum inhibitory concentration (see test example 1 below) but the compounds of the invention show, in comparison with the control, a significantly improved effect against plant pathogenic microorganisms (see test examples 3, 5 and 6 below).

In formula (I), (II), (IV) and (VI), above, Ar represents phenyl or naphthyl. Where Ar is naphthyl, the position of its bonding may be either in the $\alpha$- or the $\beta$- position. Both of these aromatic radicals may be unsubstituted, and the phenyl group may have one to five substituents, while the naphthyl may have one to seven substituents. Examples of such substituents may include alkyls having 1 to 4 carbon atoms, such as methyl, ethyl, *n*-propyl, *i*-propyl, *n*-butyl, *i*-butyl, *sec*-butyl and *t*-butyl; alkoxys having 1 to 4 carbon atoms, such as methoxy, ethoxy, *n*-propoxy, *i*-propoxy, *n*-butyoxy, *i*-butyoxy and *sec*-butyoxy; alkylthio groups having 1 to 4 carbon atoms, such as methylthio, ethylthio, *n*-propylthio, *i*-propylthio, *n*-butylthio, *i*-butylthio, *sec*-butylthio and *t*-butylthio; alkylsulfinyl groups having 1 to 4 carbon atoms, such as methylsulfinyl, ethylsulfinyl, *n*-propylsulfinyl, *i*-propylsulfinyl, *n*-butylsulfinyl, *i*-butylsulfinyl, *sec*-butylsulfinyl and *t*-butylsulfinyl; alkylsulfonyl, having 1 to 4 carbon atoms, such as methylsulfonyl, ethylsulfonyl, *n*-propylsulfonyl, *i*-propylsulfonyl, *n*-butylsulfonyl, *i*-butylsulfonyl, *sec*-butylsulfonyl and *t*-butylsulfonyl; halogen atoms such as fluorine, chlorine, bromine and iodine; nitro; trifluoromethyl; and di-$C_{1-4}$ alkylamino groups such as dimethylamino, diethylamino, methyl-ethyl-amino, methyl-*i*-propylamino, di-*n*-propylamino, di-*i*-propylamino, di-*n*-butylamino and di-*i*-butylamino. When not less than two of these substituents occur, they may be not only the same but also different substiuents which may be present in not less than two kinds mixed. Among these substituents, $C_{1-4}$ alkyl, $C_{1-4}$ alkoxy, $C_{1-4}$ alkylthio, halogen and trifluoromethyl are preferably employed, and alkyl groups such as methyl or ethyl, or halogen atoms (e.g. chlorine or bromine) are particularly preferred. As to the position of substitution, the phenyl may be substituted by any kind of substituent in any number at any position, provided the number of substituents is not more than 5, and preferably by the substituent in at least one *o*-position (2-position), whereby the particularly preferred substituents are $C_{1-4}$ alkyls such as methyl and ethyl and halogen atoms such as chlorine and bromine. As to the second to fourth substituents, the above-mentioned substituents may, or may not, enter, in any combination, any remaining positions. However, 1 to 4 as a total number of substituents is particularly preferred and, in view of the tendency for even the above-mentioned substituents, when they enter both of the *o*-positions (2,6-positions) at the same time, generally to have difficulty in forming a hydrazone linkage, it is especially desirable that the number of substituents should not be more than 4. Analogously, the same is true with $\alpha$- and $\beta$-naphthyl groups. For example, it is preferable that a substituent should be present at the 2- position in the case of $\alpha$-naphthyl and at the 1- or 3- position in the base of $\beta$-naphthyl, so that the $C_{1-4}$ alkyls and halogen atoms mentioned above are preferred as substituents of the phenyl group. Other substituents may be present at any of the remaining positions, although they preferably enter the 4- or/and 6-positions. It should be noted, however, that a total number of such substituents of 1 or 2 is especially desirable.

$R^1$ in the formulae (I), (II), (IV) and (VI) represents, for example, alkyls having 1 to 4 carbon atoms, such as methyl, ethyl, *n*-propyl, *i*-propyl, *n*-butyl, *i*-butyl, *sec*-butyl and *t*-butyl; $C_{3-5}$ cycloalkyls such as cyclopropyl and cyclopentyl; trifluoromethyl; $C_{1-4}$ alkoxycarbonyl groups, such as methoxycarbonyl and ethoxycarbonyl; phenyl and benzyl, and the phenyl may be substituted by a halogen as described above and may, for example, be *o*-, *m*- or *p*-chlorophenyl. Among these substituents, $C_{1-4}$ alkyl and $C_{3-5}$ cycloalkyl; are preferably employed, and $C_{1-4}$ alkyl such as methyl or ethyl is particularly preferred.

$R^2$ in the formulae (I), (III), (IV) and (VI) represents hydrogen or $C_{1-4}$ alkyl such as methyl, ethyl, *n*-propyl, *i*-propyl, *n*-butyl, *i*-butyl or *sec*-butyl. Among these substituents, hydrogen is especially desirable.

$R^3$, $R^4$ and $R^5$ in the formulae (I) and (III) as well as $R^{3'}$, $R^{4'}$ and $R^{5'}$ in the formulae (V) and (VI) indicate hydrogen atoms; alkyls having 1 to 4 carbon atoms, such as methyl, ethyl, *n*-propyl, *i*-propyl, *n*-butyl, *i*-butyl, *sec*-butyl and *t*-butyl; alkenyls having 2 to 4 carbon atoms, such as vinyl, allyl, crotyl and

methallyl; and alkoxy groups having 1 to 4 carbon atoms, such as methoxy, ethoxy, *n*-propoxy, *i*-propoxy, *n*-butyoxy, *i*-butoxy and *sec*-butoxy. Among these substituents, hydrogen, $C_{1-4}$ alkyl and $C_{2-4}$ alkenyl are preferably employed, and $C_{1-4}$ alkyl such as methyl or ethyl and $C_{2-4}$ alkenyl such as allyl are particularly preferred. Further, at least one of $R^3$, $R^4$ and $R^5$ or $R^{3'}$, $R^{4'}$ and $R^{5'}$ may be $C_{1-4}$ alkyl as mentioned above to obtain a good result. In other words, it is particularly preferred that $R^3$ and $R^{3'}$ by $C_{1-4}$ alkyls such as methyl or ethyl, $R^4$ and $R^{4'}$ being hydrogen or $C_{1-4}$ alkyl such as methyl or ethyl, and $R^5$ and $R^{5'}$ be $C_{1-4}$ alkyls such as methyl and ethyl. Where $R^3$, $R^4$ and $R^5$, or $R^{3'}$, $R^{4'}$ and $R^{5'}$ are $C_{1-4}$ alkyls, they may each be the same as the others or different from one another. Furthermore, $R^3$ and $R^4$ or $R^4$ and $R^5$ may be combined with each other to represent trimethylene, tetramethylene or butadienylene, while $R^{3'}$ and $R^{4'}$ or $R^{4'}$ and $R^{5'}$ may be combined with each other to represent trimethylene or tetramethylene. Thus, this means that $R^3$ and $R^4$ or $R^4$ and $R^5$, or $R^{3'}$ and $R^{4'}$ and $R^{5'}$ may be combined with each other to form a connective cross-linking bond and cooperate with two carbon atoms of the pyrimidine ring to form a saturated or unsaturated, 5- or 6-membered condensed carbon ring. When $R^3$ and $R^4$ or $R^4$ and $R^5$ are combined with each other to form a butadienylene, this means that they form a benzene ring in conjunction with two carbon atoms of the pyrimidine ring, or that the whole of the ring is a benzopyrimidine or quinazoline ring. When $R^3$ and $R^4$ or $R^4$ and $R^5$, or $R^{3'}$ and $R^{4'}$ or $R^{4'}$ and $R^{5'}$ are said to be combined with each other form a tetramethylene group, this refers to the formation of a tetrahydrobenzopyrimidine or tetrahydroquinazoline ring. Among these substituents, the butadienylene group is especially desirable.

A pyrimidine derivative (I) or a salt thereof is produced, for example, by reacting an aromatic ketone (II) with a 2-pyrimidylhydrazine (III) or a salt thereof.

In conducting the reaction of an aromatic ketone designated by the formula (II) with 2-pyrimidylhyrazine indicated by the formula (III), the latter may be subjected to the reaction, either in the free state or as a salt with an organic and inorganic acid. As the organic acid, formic acid, acetic acid and propionic acid, for example, are employable, and, as the inorganic acid hydrochloric acid, sulfuric acid, and phosphoric acid, for example, are employable. The reaction may be carried out by mixing of the two compounds (II) and (III) or a salt thereof in about equimolar amount, although a slight excess of either of the two may be charged to the reaction mixture. When the aromatic ketone (II) is liquid, for example, it may be used in excess to allow it to act as a solvent as well. Furthermore, when both of the compounds are solid, they may be melted into the liquid state by heating. In order to allow the reaction to proceed smoothly, however, the reaction is preferably carried out in an organic solvent, whereby the organic solvent may be any type of solvent, unless the solvent would exert an adverse effect upon the reaction. Alcohols such as methanol, ethanol, propanol or butanol, for example, and methyl cellosolve or ethyl cellosolve, ethers such as diethyl ether, tetrahydrofuran, dioxane or dimethoxyethane and aromatic hydrocarbons such as benzene, toluene or xylene are particularly preferred. These solvents may be used, alone or in various mixtures of two or more of them in varying mixing ratios.

The reaction, generally, proceeds smoothly. Therefore, heating is not always required, when sufficient stirring or shaking is used, but is sometimes required, if the completion of the reaction within a short period of time is desired. The reaction temperature, normally, is desirably in the range of 30°C to 150°C, although a higher temperature near to 200°C is in some instances required. Normally, the reaction is conducted at atmospheric pressure and, in some cases, can be carried out under elevated pressure applied with the use of a tightly sealed container. The reaction time, which varies with the kinds of starting materials and solvents and the reaction temperature, goes ordinarily to completion within from several tens of minutes to several hours, and yet, in some instances extends to several tens of hours.

This reaction involves essentially the formation of hydrazones through a dehydration condensation reaction of ketones with hydrazines, whereby no particular attention need be paid to moisture removal or dehydration of the reaction system under normal reaction conditions. In cases where acceleration of the reaction rate or enhancement of the yield is desired, however, satisfactory results may in some instances by obtained by employing both starting materials and solvent adequately dried and dehydrated, and paying attention to preventing the moisture from entering during the reaction, and taking water produced in the reaction system out of the system through azeotropic distillation or adding a dehydrating agent such as a molecular sieve to the reaction system.

Although the presence of a catalyst in this reaction is not essential, the addition of traces of an acid or a base results, in some cases, a marked acceleration of the rate of reaction. Such acid may be either an organic or an inorganic acid. As the organic acid, formic acid, acetic acid or propionic acid, which may be made also serve as solvents are employable; for instance; examples of an inorganic acid which is employable include hydrochloric acid, sulfuric acid, phosphoric acid and polyphosphoric acid (PPA), polyphosphoric acid ester (PPE), titanium tetrachloride, boron trifluoride, and other Lewis acids; among these, sulfuric acid or polyphosphoric acid (PVA) may, e.g., be made to serve both as a solvent and as a dehydrating agent. The bases which are usable include, for example, inorganic bases such as potassium hydroxide, sodium hydroxide and sodium alcoholate, and organic bases such as pyridine and triethylamine, of which the latter may be made to serve as a solvent. In addition, acidic or basic ionexchange resins may be employed as solid catalysts. Furthermore, when hydrazine (III) as an acid salt is subjected to reaction as mentioned above, this means that the acid is introduced into the reaction

system as a catalyst. Generally speaking, it is the acids which can produce, as the catalyst, the more desirable results in terms of the rate of reaction, the yield, the colouration, and so on.

A pyrimidine derivative (VI) or a salt thereof may also be produced by reacting amidinohydrazone (IV) or a salt thereof with β-diketone (V).

In the reaction of an amidinohydrazone represented by the formula (IV) with a β-diketone designated by the formula (V), the former may be subjected to the reaction, either in the free state or as an acid salt with such an organic or inorganic acid as mentioned hereinbefore. This reaction is conducted by mixing nearly equimolar amounts of both the compound (IV) or a salt thereof and the compound (V), although either of compounds IV (or its salt) and (V) may in some casese be charged in slightly excess. Where the β-diketone (V) is liquid, for example, it may be employed in excess so as to serve also as a solvent. Where both are in the solid state, they may be melted and liquefied by heating. However this reaction is preferably conducted in an organic solvent so as to allow it to proceed smoothly, and the organic solvent may be any of various type of solvent, provided it does not adversely affect the reaction; for example, alcohols, ethers or aromatic hydrocarbons, as mentioned above in connection with the reaction of (II) and (III), are particularly preferred. These solvents may be used, alone or as a mixture of two or more kinds thereof in different mixing ratios.

The reaction generally proceeds smoothly. Therefore, heating is not always required, when sufficient stirring or shaking is used, but it sometimes employed if the completion of the reaction within a short period of time is desired. The reaction temperature is normally in the range of from 40°C to 200°C, and desirably kept especially within the range of from 60°C to 150°C. Ordinarily, the reaction is conducted at atmospheric pressure and, in some instances, may be carried out under elevated pressure applied with the use of a tightly sealed container. The reaction time, which varies with the kinds of starting materials and solvents and the reaction temperature, goes normally to completion within from several tens of minutes to some hours and, in some cases, extends to several tens of hours.

The reaction involves essentially the formation of a pyrimidine ring through the dehydration condensation reaction of an amidine with a β-diketone, and, as to the dehydrating conditions and the catalyst effects in the reaction system, substantially the same criteria as are described in the reaction between (II) and (III), or salts thereof, are applicable.

The end point of the reaction between (II) and (III), or salts thereof, or between (IV) (or a salt thereof) and (V) may be easily ascertained by thin layer chromatography, for example. Thus, the reaction may be completed at the time when a spot other than those of starting materials becomes detectable on thin-layer silica gel by ultraviolet irradiation (2536 Å) or sprayed Dragendoff reagent.

The pyrimidine derivatives (I), or salts thereof produced in this manner, are novel compounds which have not previously been described in the literature. These derivatives are normally, at room temperature, colourless or slightly coloured, crystalline solids or viscous oils, and present a starch-syrup-like or glass-like semi-solid state, when they are highly viscous. Generally, they are substantially insoluble in water but readily soluble in various organic solvents, for example, alcohols, ethers and aromatic hydrocarbons being employed in the reaction as well as aliphatic halogenated hydrocarbons such as chloroform and methylene chloride, esters such as ethyl acetate and butyl acetate, acid amides and nitriles such as dimethylformamide and acetonitrile, and the like. Consequently, when the derivative is a crystalline solid, after the completion of the reaction, the reaction mixture may be directly cooled, or admixed with water in the case of the reaction solvent being miscible with water, or freed of the reaction solvent, and the resulting crude product is recrystallized from an appropriate solvent. When it is an oily substance, the crude product obtained by a similar treatment is purified by the use of column chromatography. In cases in which an acidic or basic catalyst is added, or an acidic or basic solvent is employed, a neutralization treatment must be carried out in accordance with the nature of the solution. When an acidic catalyst or an acidic solvent is used, the above-mentioned treatment may be directly carried out to isolate the reaction product as an acid salt. Further, the reaction product, once having been isolated as a free base, may, if desired, be converted into a salt with a variety of organic or inorganic acids as mentioned above in connection with 2-pyrimidylhydrazine (III), and the resulting acid salts, together with the free base, are included within the definition of the desired compound (I). The structure of a reaction product may be confirmed by elementary analysis, infra-red absorption spectra, ultra-violet absorption spectra, mass spectra or nuclear magnetic resonance spectra, for example.

The pyrimidine derivative (I), or a salt thereof, a reaction product of the above-mentioned reaction, is a kind of hydrazone compound having a C=N double bond in the molecule, and, consequently, exists in two geometrical isomers, Z and E types, in relation to this bond. For example, there is often observed the formation of two isomers as two adjacent spots on the thin layer chromatogram obtained with a crude reaction product. Yet, the proportion of two isomers varies depending upon the kinds of starting materials and solvent, the reaction conditions (temperature and duration time), the acidity of the solution, the type of catalysts and whether or not they are added, and a single isomer alone may be produced, as the case may be. In cases where the isomers are produced as a mixture, they may be isolated by carrying out through purification by means of column chromatography, for example. In this case, it often occurs that elution with chloroform elutes the Z-isomer faster, while elution with ethyl acetate elutes the E-isomer more quickly. As to the identification of the isomers, each of them may be discriminated by chemical shifts of proton signals in the NH group of the molecule in the nuclear

magnetic resonance spectra. In other words, the chemical shift for the E-isomer is in many cases located relatively at a lower magnetic field than that for the Z-isomer. Consequently, the isomer ratio of the mixture may be determined by the integrated intensity ratio for each of the peaks. However, these isomers are tautomeric being vunerable to isomerization by heating and light irradiation, and it is therefore useless forcibly to isolate the mixture of isomers to each isomer where the isolation is difficult, while there is no adverse effect in subjecting the mixture of isomers to the application fields of the present invention.

Referring now to the starting materials to be used in the above-mentioned reactions, the aromatic ketone represented by the formula (II), as described on the lists of various reagent makers in Japan and elsewhere, is readily available in many kinds. The others may be readily synthesized by conventional methods of aromatic ketone synthesis, for example, by general synthesis methods such as the Friedel-Crafts acylation of aromatic hydrocarbons with carboxylic acids or their derivatives, and methods in accordance with such reaction, [see, e.g.: G. A. Olah (Editor), "Friedel-Crafts and Related Reactions", Vol. III (Part 1), 1 (1964); Chemical Society of Japan, "Zikkenkagakukoza (Course of Experimental Chemistry)", Vol. 19, 316 (1957); and Chemical Society of Japan, "Sin-Zikkenkagakukoza (New Course of Experimental Chemistry), Vol. 14 (II), 751 (1977)], as well as the methods described, e.g. in Journal of Organic Chemistry, *11*, 444 (1946); *ibid., 12*, 617 (1947); *ibid., 31*, 1655 (1966); Journal of the Chemical Society, *1952*, 1123, 4162; *ibid., 1955*, 3417; *ibid., 1968C*, 2502; *ibid., 1971C* 3347; and the Canadian Journal of Chemistry, *41*, 2103 (1963), and methods in accordance therewith.

The following Table 1 tabulates physical constants or the appearance of some novel compounds out of the aromatic ketones (II).

TABLE 1

$ArCOR^1$

| Ar | $R^1$ | Physical constants or appearance |
|---|---|---|
| 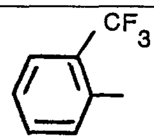 | $CH_3$ | b.p., 89 °C/15 mmHg |
| 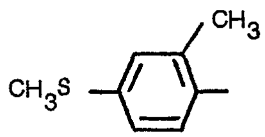 | $CH_3$ | b.p., 108–110 °C/0.2 mmHg |
| 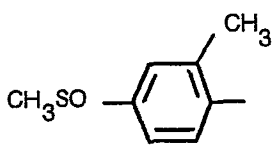 | $CH_3$ | Oily substance |
| 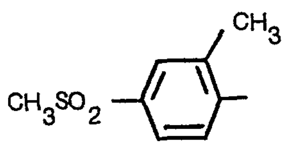 | $CH_3$ | Oily substance |
| 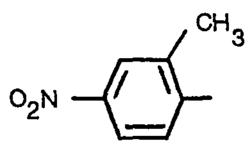 | $CH_3$ | b.p., 73 °C |
| 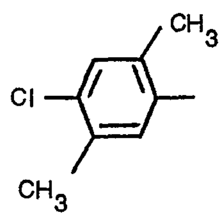 | $CH_3$ | Solid substance |
| 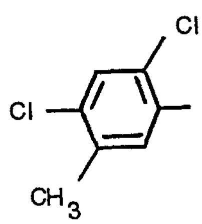 | $CH_3$ | b.p., 150–152 °C/20 mmHg |
| 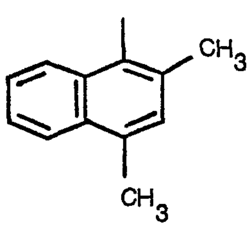 | $CH_3$ | Solid substance |

As to the 2-pyrimidylhydrazine represented by the formula (III), many homologues are known in the literature and the others may be synthesized by the known methods described in the literature or methods in accordance therewith. For example, they may be easily produced by the reaction with hydrazine or a mono-lower-alkylhydrazine such as methylhydrazine or ethylhydrazine, in the presence of an organic or inorganic base, of a pyrimidine derivative having, in the 2-position, a halogen atom such as chlorine or bromine, a lower alkoxy group such as methoxy or ethoxy, a phenoxyl group, a mercapto group, a lower alkylthio group such as methylthio or ethylthio, a phenylthio group, a lower alkylsulfonyl group such as methylsulfonyl or ethylsulfonyl, a phenylsulfonyl group, a nitroamino group, a cyanoamino group or a tri-lower-alkylammonium group such as trimethylammonium or triethylammonium [see e.g. Yakugaku Zasshi, *73,* 159 and 598 (1953); *ibid., 79,* 1477 (1959); Chemical and Pharmaceutical Bulletin, *17,* 1479 (1969); and Australian Journal of Chemistry, *30,* 2515 (1977)].

The following Table II shows the melting points of two novel compounds of 2-pyrimidylhydrazine (III):

TABLE II

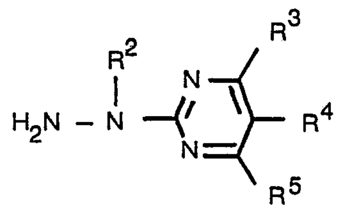

| $R^2$ | $R^3$ | $R^4$ | $R^5$ | Physical constants |
|---|---|---|---|---|
| H | $CH_3$ | $CH_3$ | $CH_3$ | m.p. 165 – 167 °C. |
| H | $CH_3$ | $-(CH=CH)_2-$ | | m.p. 180 – 181 °C. |

As the amidinohydrazone represented by the formula (IV), the non-substituted homologue ($Ar=C_6H_5$; $R^1=CH_3$; $R^2=H$) is the known compound described in the literature and the others may be produced by the known methods described in the literature or methods in accordance therewith. For example, they may be easily synthesized by reacting an aromatic ketone (II) with an aminoguanidine bicarbonate salt or nitrate salt in the presence of an organic or inorganic base. The amidinohydrazone obtained in this manner has the basicity to be isolatable as a salt of an organic or inorganic acid, not to mention as a free base. The acid salt may be freshly neutralized so as to be subjected to the reaction according to the present invention as a free base, or subjected to the reaction as an acid salt [Annalen der Chemie, *307,* 293 (1899)]. Although it is expected that the Z-form and E-form geographical isomers may exist in relation to the C=N bond of the amidino-hydrazone, the mixture of isomers without isolation for identification or either of these without being characterized may be subjected to the reaction according to the present invention.

The following Table III shows the melting points of these novel compounds of the amidinohydrazone (IV):

TABLE III

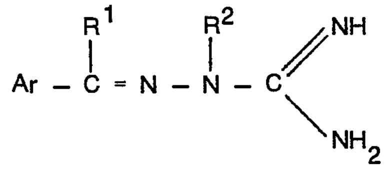

| Ar | $R^1$ | $R^2$ | Physical constants |
|---|---|---|---|
| 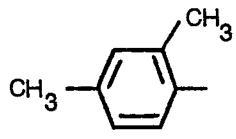 | $CH_3$ | H | m.p. about 160°C |
| 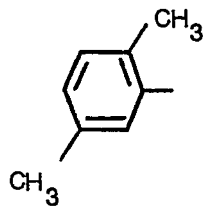 | $CH_3$ | H | m.p. about 125°C |
| 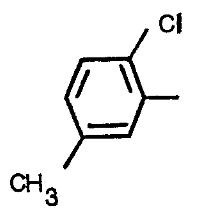 | $CH_3$ | H | m.p. about 174°C (acetate) |

β-diketones represented by the formula (V) are described in the lists of reagent makers in Japan and elsewhere and are readily available in various kinds. The others may be easily synthesized by the ordinary methods of synthesis of β-diketones, for example, the general method based on the alkylation reaction of acetylacetone, or methods in accordance therewith [see e.g.: H. O. House, "Modern Synthetic Reactions", 2nd ed., 492 and 734 (1972); Chemical Society of Japan (editor), "Zikkenkagakukoza (Course of Experimental Chemistry)", *19*, 316 (1957); and Chemical Society of Japan, "Sin-Zikkenkagakukoza (New Course of Experimental Chemistry)" *14*(II), 751 (1977)], as well as by the methods described e.g., in Organic Syntheses, III, 291 (1955); *ibid.,* V. 785 and 848 (1973); Chemical Bulletin of Japan, *88* 1068 (1967); and Journal of the American Chemical Society, *68*, 453 (1946), and methods in accordance therewith.

The pyrimidine derivatives (I) or salts thereof according to the present invention possess a strong antimicrobial acitivity against a wide range of plant pathogenic microorganisms, particularly against fungi, and, when they are applied as an antimicrobial agent for paddy field uses, not only exterminate *Pyricularia oryzae* Cavara but also possess the exterminating effect against *Pellicularia sasakii* (Shirai) S. Ito, *Helminthosporium sigmoideum* and *Helminthosporium oryzae,* for example. Furthermore, they have a strong antimicrobial activity against not only pathogenic microorganisms of rice plants but also those causing diseases on vegetables and many other crops. For example, they have an antimicrobial activity against *Phytophthora capsici, Sclerotinia sclerotiorum* (Libert) deBary and *Botrytis cinerea.*

In addition, the compounds (I) or salts thereof according to the present invention possess not only the therapeutic capacity of acting, when applied to an already disease-attacked plant, to inhibit the disease expansion, but also the preventive capacity of preventing when applied to unattacked plants, the infection by a pathogenic agent to protect such plant. As to the application method, they may be applied by spraying to the stems and leaves of plants and applied to the root portions of plants, whereby they, with their strong penetrating property are absorbed into the plants, migrating through them to spread widely, and develop the capacity of retaining the concentration necessary for protecting the plants.

The compounds (I) or salts of them, despite their strong antimicrobial activities, are low in skin irritating property and oral toxicity towards warm-blooded animals, and exercise a reduced effect on the environment, for example in terms of fish toxicity, etc. Furthermore, they exhibit a phytotoxic action against a variety of plants, which is non-resistant or merely slight, and affect in no way subsequent growths and crop yields. This may be ascribed to the fact that the compounds (I) or their salts have a strong affinity towards plants and a proper degree of chemical stability. In other words, they are

assumed to be gradually inactivated through hydrolysis of the hydrazone linkage contained in the molecules. It may be said, consequently, that the pyrimidine derivatives (I) or their salts according to the present invention are provided with a highly superior nature and properties as antimicrobial agents for multi-purpose, agricultural uses.

The antimicrobial agent according to the present invention may consist of two or more kinds of the compounds of the formula (I) or salts of them in combination, not to mention one kind thereof. The antimicrobial agent may comprise a free base of, or an organic or inorganic acid salt of, the compounds (I) or salts of them of the present invention (hereinafter referred to as the active component), alone or in conjunction with a variety of natural materials, additives, solvents, etc. being added, as occasion demands. Referring more particularly to this, the active component may be used as a solid, as it is, for the purpose of retaining its effectiveness for a prolonged period of time, or may be dissolved or dispersed in a suitable liquid carrier (for example, a solvent), or admixed with or absorbed on an appropriate solid carrier (for example, a diluent or an extender), followed adding an emulsifying agent, dispersing agent, suspending agent, spreader, penetrant, wetting agent, thickening agent, stabilizer, for use as an oil preparation, emulsifiable concentrate, wettable powder, aqueous solution, suspension, dust, granules, fine granules, tablet, spray, or other suitable preparation forms.

Water alcohols (e.g. methyl alcohol, ethyl alcohol, ethyleneglycol, propyleneglycol), ketones (e.g. acetone, methyl ethyl ketone), ethers (e.g., dioxane, tetrahydrofurane, cellosolve), aliphatic hydrocarbons (e.g., gasoline, kerosene, light oil, fuel oil, machine oil), aromatic hydrocarbons (e.g., benzene, toluene, xylene, solvent naphtha, methylnaphthalene) and other organic bases (e.g., pyridine, aldehyde collidine), halogenated hydrocarbons (e.g., chloroform, carbon tetrachloride), acid amides (e.g., dimethylformamide), esters (e.g., ethyl acetate, butyl acetate, glycerine esters of fatty acids,) and nitriles (e.g., acetonitrile), and sulfur-containing compounds (e.g., dimethylsulfoxide, tetramethylene sulfone), and the like, may be used as such solvents.

The solid carrier such as the diluent or extender may be, for example, a powder of plant origin (e.g., rice bran, soybean powder, tobacco powder, wheat flour, wood powder), a powder of mineral origin (e.g., kaolin, bentonite, calcium phosphate, clays such as acid clay, talcs such as talc powder and pagotite powder, silicas such as diatomaceous earth and mica powder), and alumina, sulfur powder or activated carbon, which may be used alone or as a mixture of not less than two kinds.

The emulsifying agent, spreader, penetrant, dispersing agent and the like which is used may include soaps, sulfates of higher alcohols, alkyl sulfonic acids, alkyl aryl sulfonic acids, quarternary ammonium salts, oxyalkylamines, fatty acid esters, surface active agents based on polyalkylene oxide, anhydrosorbitol, which are preferably incorporated into preparations, generally at a level of 0.2 to 10%. Further, casein, gelatin, starch, arginic acid, agar, CMC, polyvinyl alcohol, pine oil, rice bran oil, bentonite, cresol soap, may be used, if desired. In addition, there may be suitably admixed therewith, as occasion demands, various different kinds of fungicides and bactericides (e.g., organic chlorine fungicides, organic phosphorus fungicides, benzimidazole fungicides, copper fungicides, organic sulfur fungicides, phenol fungicides, antibiotics), insecticides, (e.g., natural insecticides, carbamate insecticides, organic phosphorus insecticides) and others such as miticides, nematocides, herbicides, plant growth regulators, stabilizers, synergists, attractants, repellent, perfumes, plant nutrients, fertilizers, amino acids and low-molecular or high-molecular-weight phosphoric acid salts, for example, while metal salts may be added for the purpose of strengthening the effectiveness of the preparation.

The content of the active component in the antimicrobial agents for control uses according to the present invention may suitably be in the range of 10 to 90% for emulsifiable concentrate, wettable powder, 0.1 to 10% for oil preparation, dust, and 5 to 50% for granules.

Meanwhile, the emulsifiable concentrate, wettable powder, etc., when brought into practical use, may be suitable diluted with water, etc. (for example, up to 50 to 5000 times) so as to be sprayed.

The amount of the active component mixture thereof with other kinds of antimicrobial agents and the formulation ratio vary depending upon the growth phase of the plant to be treated, its growth condition, the species of disease, the condition of the disorder, the application time or method for the antimicrobial agent, and other conditions, and is generally adjusted in such a way that the active component is used at an application rate within the range of from 10 to 300 g per 10 are. The application concentration may be in the range of 10 to 1000 ppm of the active component, while the application method may be by means of spraying, dusting and irrigating on crops or dust coating of seeds; any application method provided safe and effective application to crops is secured, we do not impose any restriction on the present invention, no matter what the used amount, the application concentration and the application method may be.

The antimicrobial agent for the plant disease controlling uses according to the present invention has reduced sideeffects and can achieve a superior action and effectiveness by a simple procedure, at reduced cost, and to a precise degree, thus offering a high level of usefulness in commercial use.

In the specification, the following abbreviations are used: "ml" = milliliter, "mM" = millimol, "mg" = milligram, "g" = gram, "μg" = microgram, "mm" = millimeter, "cm" = centimeter, "a" = are, "%" = percent, "NMR" = Nuclear Magnetic Resonance, "S" = singlet, "ppm" = part per million, "comp." = Compound, "No." = Number, "Synth." = Synthesis, "Phys." = Physical, "ca" = circa, "m.p." = melting point, "Concn." = Concentration.

The Examples and Test Examples are as follows:

Example 1

To 15 ml of ethanol are added 1.50 g (9mM) of *o*-methyl-thioacetophenone (11: Ar=o-$CH_3S.C_6H_4$; $R^1$=$CH_3$) and 1.38 g (10mM) of 2-hydrazino-4,6-dimethylpyrimidyl (111: $R^2$=$R^4$=H; $R^3$=$R^5$=$CH_3$), followed by boiling under reflux for about 13 hours. After the reaction, the reaction mixture is concentrated under reduced pressure, and the resultant viscous, oily substance is chromatographed on a column (silica gel/chloroform). Since the Z-form of 4,6-dimethyl-2-[1-(2-methyl-thiophenyl)ethylidenehydrazino]pyrimidine is eluted first, together with the E-form, after a short time interval, elution of both isomers with chloroform yields the elution fractions containing the same isomer which are collected by checking with thin-layer chromatography, followed by concentration of each of them to yield the isomers as a crystalline solid, respectively.

*Z-form*

Yield: 0.95 g (38%). Melting point: 125—127°C

Elementary analysis ($C_{15}H_{18}N_4S$)

| | C | H | N |
|---|---|---|---|
| Calcd. (%) | 62.90 | 6.33 | 19.56 |
| Found (%) | 62.80 | 6.40 | 19.59 |

NMR ($CDCl_3$, ppm), $\delta$ value

Pyrimidine 4,6-$CH_3$: 2.33 (6H, s.), N=C—$CH_3$,$SCH_3$: 2.35 (3H, s.), 2.43 (3H, s.)

Pyrimidine 5-H: 6.46 (1H, s.), phenyl proton: 7.0—7.5 (4H, m.), NH: 7.88 (1H, s.).

*E-form:*

Yield: 1.20 g (48%), melting point: 94°C

Elementary analysis ($C_{15}H_{18}N_4S$)

| | C | H | N |
|---|---|---|---|
| Calcd. (%) | 62.90 | 6.33 | 19.56 |
| Found (%) | 62.85 | 6.23 | 19.46 |

NMR ($CDCl_3$, ppm), $\delta$ value

Pyrimidine 4,6-$CH_3$: 2.35 (6H, s.), N=C—$CH_3$, $SCH_3$: 2.30 (3H, s.), 2.41 (3H, s.)

Pyrimidine 5-H: 6.51 (1H, s.), phenyl proton: 7.0—7.5 (4H, m.), NH: 8.25 (1H, s.)

Example 2

A 2.00 g (10mM) portion of 2-(2,4-dimethylphenyl)ethylideneaminoguanidine (1V: Ar=2.4-$(CH_3)_2C_6H_3$; $R^1$=$CH_3$; $R^2$=H) and 2.90 g (23mM) of 3-ethylacetylacetone (V: $R^3$=$R^5$=$CH_3$; $R^4$=$C_2H_5$) are stirred for about 3.5 hours, while keeping them at 130 to 140°C in an oil bath. After the reaction, $\beta$-diketone excess is distilled off under reduced pressure, and the resulting viscous, oily substance is chromatographed on a column (silica gel/ethyl acetate + *n*-hexane). Since the Z-form of 5-ethyl-4,6-dimethyl-2-[1-(2,4-dimethylphenyl)ethylidenehydrazino]-pyrimidine is eluted first, with the E-form successively eluted partly in a mixture with the Z-form, by elution of both isomers with a mixed solvent of ethyl acetate and *n*-hexane, the elution fractions containing the same isomer are collected by checking through thin-layer chromatography, followed by concentration of each of them to give the Z-form as a crystalline solid and the E-form as a viscous oily substance.

*Z-form:*

Yield: 1.50 g (51%), melting point: 127—129°C

Elementary analysis ($C_{18}H_{24}N_4$)

| | C | H | N |
|---|---|---|---|
| Calcd. (%) | 72.93 | 8.16 | 18.90 |
| Found (%) | 72.35 | 8.09 | 18.72 |

NMR ($CDCl_3$, ppm), $\delta$ value

NH: 7.72 (1H, s.)

*E-form (mixed with Z-form):*

Yield: 0.70 g (24%). Viscous, oily substance

Elementary analysis ($C_{18}H_{24}N_4$)

| | C | H | N |
|---|---|---|---|
| Calcd. (%) | 72.93 | 8.16 | 18.90 |
| Found (%) | 72.78 | 7.99 | 18.48 |

NMR ($CDCl_3$, ppm), $\delta$ value

NH: 7.75 and 8.08 (ratio of Z-form: E-form = 1:2)

Example 3

The compounds Nos. 1 to 126, as described hereinafter are produced in the same manner as in Example 1 (Synthesis method A) or in Example 2 (Synthesis method B). Their chemical structures (Ar, $R^1$, $R^2$, $R^3$, $R^4$, $R^5$), the method of synthesis and the physical constants (appearance), of these compounds inclusive of the compounds obtained in Examples 1 and 2, are tabulated in the following Table. In the column (ratio of Z:E) of the Table, E stands for the E-form solely obtained and Z for the Z-form alone, while Z + E stands for a mixture of both isomers, with their ratio unidentified; the compound No. 115, with Ar and R′ being the same, has no structural isomer and can be isolated by merely concentrating after the reaction.

| Comp. No. | Ar | $R^1$ | $R^2$ | $R^3$ | $R^4$ | $R^5$ | Ratio of Z : E | Synth. method | Phys. constants (appearance) |
|---|---|---|---|---|---|---|---|---|---|
| 1 | $C_6H_5$ | $CH_3$ | H | $CH_3$ | H | $CH_3$ | E | A | m.p. 89—91°C |
| 2 | O-$CH_3$·$C_6H_4$ | $CH_3$ | H | $CH_3$ | H | $CH_3$ | E | A | m.p. 178—180°C |
| 3 | O-$CH_3$·$C_6H_4$ | $CH_3$ | H | $CH_3$ | H | $CH_3$ | E | A | m.p. 110—111°C |
| 4 | *m*-$CH_3$·$C_6H_4$ | $CH_3$ | H | $CH_3$ | H | $CH_3$ | E | A | m.p. 85—88°C |
| 5 | *p*-$CH_3$·$C_6H_4$ | $CH_3$ | H | $CH_3$ | H | $CH_3$ | E | A | m.p. 164—166°C |
| 6 | O-$CH_3O$·$C_6H_4$ | $CH_3$ | H | $CH_3$ | H | $CH_3$ | E | A | m.p. 128°C |
| 7 | O-$CH_3S$·$C_6H_4$ | $CH_3$ | H | $CH_3$ | H | $CH_3$ | E | A | m.p. 125—127°C |
| 8 | O-$CH_3S$·$C_6H_4$ | $CH_3$ | H | $CH_3$ | H | $CH_3$ | E | A | m.p. 94°C |
| 9 | O-Cl·$C_6H_4$ | $CH_3$ | H | $CH_3$ | H | $CH_3$ | E | A | m.p. 132°C |
| 10 | *m*-Cl·$C_6H_4$ | $CH_3$ | H | $CH_3$ | H | $CH_3$ | E | A | m.p. 110°C |
| 11 | *p*-Cl·$C_6H_4$ | $CH_3$ | H | $CH_3$ | H | $CH_3$ | E | A | m.p. 166—168°C |
| 12 | O-F·$C_6H_4$ | $CH_3$ | H | $CH_3$ | H | $CH_3$ | E | A | m.p. 132—134°C |
| 13 | O-Br·$C_6H_4$ | $CH_3$ | H | $CH_3$ | H | $CH_3$ | E | A | m.p. 129—131°C |
| 14 | O-I·$C_6H_4$ | $CH_3$ | H | $CH_3$ | H | $CH_3$ | E | A | m.p. 138—139°C |
| 15 | O-$NO_2$·$C_6H_4$ | $CH_3$ | H | $CH_3$ | H | $CH_3$ | E | A | m.p. 144—145°C |
| 16 | O-$CF_3$·$C_6H_4$ | $CH_3$ | H | $CH_3$ | H | $CH_3$ | E | A | m.p. 123—125°C |
| 17 | 2,4-$(CH_3)_2$·$C_6H_3$ | $CH_3$ | H | $CH_3$ | H | $CH_3$ | Z | A | m.p. ca. 99°C |
| 18 | 2,4-$(CH_3)_2$·$C_6H_3$ | $CH_3$ | H | $CH_3$ | H | $CH_3$ | 3:5 | A | (viscous oil) |
| 19 | 2,5-$(CH_3)_2$·$C_6H_3$ | $CH_3$ | H | $CH_3$ | H | $CH_3$ | Z | A | m.p. ca. 99°C |
| 20 | 2,5-$(CH_3)_2$·$C_6H_3$ | $CH_3$ | H | $CH_3$ | H | $CH_3$ | 3:5 | A | (viscous oil) |
| 21 | 3,4-$(CH_3)_2$·$C_6H_3$ | $CH_3$ | H | $CH_3$ | H | $CH_3$ | E | A | m.p. 144—146°C |
| 22 | 2,4-$(Et)_2$·$C_6H_3$ | $CH_3$ | H | $CH_3$ | H | $CH_3$ | 2:1 | A | (viscous oil) |
| 23 | 2,5-$(Et)_2$·$C_6H_3$ | $CH_3$ | H | $CH_3$ | H | $CH_3$ | 1:2 | A | (viscous oil) |
| 24 | 2,4-$(i\text{-}C_3H_7)_2$·$C_6H_3$ | $CH_3$ | H | $CH_3$ | H | $CH_3$ | 2:3 | A | (viscous oil) |
| 25 | 2,5-$(i\text{-}C_3H_7)_2$·$C_6H_3$ | $CH_3$ | H | $CH_3$ | H | $CH_3$ | 1:1 | A | (viscous oil) |

| Comp. No. | Ar | $R^1$ | $R^2$ | $R^3$ | $R^4$ | $R^5$ | Ratio of Z : E | Synth. method | Phys. constants (appearance) |
|---|---|---|---|---|---|---|---|---|---|
| 26 | $2\text{-}CH_3\text{-}5\text{-}Et\cdot C_6H_3$ | $CH_3$ | H | $CH_3$ | H | $CH_3$ | 1:1 | A | (viscous oil) |
| 27 | $2\text{-}CH_3\text{-}5\text{-}n\text{-}C_3H_7\cdot C_6H_3$ | $CH_3$ | H | $CH_3$ | H | $CH_3$ | 1:1 | A | (viscous oil) |
| 28 | $2\text{-}CH_3\text{-}5\text{-}i\text{-}C_3H_7\cdot C_6H_3$ | $CH_3$ | H | $CH_3$ | H | $CH_3$ | 3:1 | A | (viscous oil) |
| 29 | $2\text{-}CH_3\text{-}5\text{-}t\text{-}C_4H_9\cdot C_6H_3$ | $CH_3$ | H | $CH_3$ | H | $CH_3$ | 1:2 | A | (viscous oil) |
| 30 | $2,4\text{-}Cl_2\cdot C_6H_3$ | $CH_3$ | H | $CH_3$ | H | $CH_3$ | E | A | m.p. 175—176.5°C |
| 31 | $2,5\text{-}Cl_2\cdot C_6H_3$ | $CH_3$ | H | $CH_3$ | H | $CH_3$ | E | A | m.p. 165—166°C |
| 32 | $3,4\text{-}Cl_2\cdot C_6H_3$ | $CH_3$ | H | $CH_3$ | H | $CH_3$ | E | A | m.p. 148—149°C |
| 33 | $2\text{-}CH_3\text{-}4\text{-}CH_3O\cdot C_6H_3$ | $CH_3$ | H | $CH_3$ | H | $CH_3$ | 1:1 | A | (viscous oil) |
| 34 | $2\text{-}CH_3\text{-}4\text{-}CH_3S\cdot C_6H_3$ | $CH_3$ | H | $CH_3$ | H | $CH_3$ | 3:1 | A | (viscous oil) |
| 35 | $2\text{-}CH_3\text{-}4\text{-}CH_3SO\cdot C_6H_3$ | $CH_3$ | H | $CH_3$ | H | $CH_3$ | 3:1 | A | m.p. 135°C |
| 36 | $2\text{-}CH_3\text{-}4\text{-}CH_3SO_2\cdot C_6H_3$ | $CH_3$ | H | $CH_3$ | H | $CH_3$ | E | A | m.p. ca. 210°C |
| 37 | $2\text{-}CH_3\text{-}4\text{-}Cl\cdot C_6H_3$ | $CH_3$ | H | $CH_3$ | H | $CH_3$ | E | A | m.p. ca. 158°C |
| 38 | $2\text{-}CH_3\text{-}4\text{-}Cl\cdot C_6H_3$ | $CH_3$ | H | $CH_3$ | H | $CH_3$ | 1:1 | A | (viscous oil) |
| 39 | $2\text{-}CH_3\text{-}4\text{-}NO_2\cdot C_6H_3$ | $CH_3$ | H | $CH_3$ | H | $CH_3$ | E | A | m.p. 170—172°C |
| 40 | $2\text{-}Cl\text{-}5\text{-}CH_3\cdot C_6H_3$ | $CH_3$ | H | $CH_3$ | H | $CH_3$ | E | A | m.p. 155—157°C |
| 41 | $2\text{-}Cl\text{-}5\text{-}CH_3\cdot C_6H_3$ | $CH_3$ | H | $CH_3$ | H | $CH_3$ | 6:1 | A | m.p. ca. 125°C |
| 42 | $2\text{-}Cl\text{-}5\text{-}CH_3O\cdot C_6H_3$ | $CH_3$ | H | $CH_3$ | H | $CH_3$ | 1:2 | A | m.p. ca. 98°C |
| 43 | $2\text{-}Br\text{-}5\text{-}CH_3\cdot C_6H_3$ | $CH_3$ | H | $CH_3$ | H | $CH_3$ | 3:4 | A | m.p. ca. 110°C |
| 44 | $2\text{-}Br\text{-}5\text{-}CH_3O\cdot C_6H_3$ | $CH_3$ | H | $CH_3$ | H | $CH_3$ | E | A | m.p. ca. 142°C |
| 45 | $2,4,5\text{-}(CH_3)_3\cdot C_6H_2$ | $CH_3$ | H | $CH_3$ | H | $CH_3$ | Z | A | m.p. ca. 112°C |
| 46 | $2,4,5\text{-}(CH_3)_3\cdot C_6H_2$ | $CH_3$ | H | $CH_3$ | H | $CH_3$ | E | A | m.p. 159—160°C |
| 47 | $2,3,4\text{-}Cl_3\cdot C_6H_2$ | $CH_3$ | H | $CH_3$ | H | $CH_3$ | E | A | m.p. 225°C |
| 48 | $2,4\text{-}(CH_3)_2\text{-}5\text{-}Et.\cdot C_6H_2$ | $CH_3$ | H | $CH_3$ | H | $CH_3$ | 2:1 | A | (viscous oil) |
| 49 | $2,5\text{-}(CH_3)_2\text{-}4\text{-}Cl\cdot C_6H_2$ | $CH_3$ | H | $CH_3$ | H | $CH_3$ | Z | A | m.p. ca. 130°C |
| 50 | $2\text{-}CH_3\text{-}4,5\text{-}Cl_2\cdot C_6H_2$ | $CH_3$ | H | $CH_3$ | H | $CH_3$ | 5:1 | A | m.p. ca. 150°C |
| 51 | $2,4\text{-}Cl_2\text{-}5\text{-}CH_3\cdot C_6H_2$ | $CH_3$ | H | $CH_3$ | H | $CH_3$ | E | A | m.p. ca. 204—205°C |
| 52 | $2,4\text{-}Cl_2\text{-}5\text{-}CH_3\cdot C_6H_2$ | $CH_3$ | H | $CH_3$ | H | $CH_3$ | 2:5 | A | m.p. ca. 196°C |
| 53 | $2,3,4,5\text{-}(CH_3)_4\cdot C_6H$ | $CH_3$ | H | $CH_3$ | H | $CH_3$ | 5:3 | A | m.p. ca. 116°C |
| 54 | $\alpha\text{-}C_{10}H_7$ | $CH_3$ | H | $CH_3$ | H | $CH_3$ | E | A | m.p. 161—163°C |

| Comp. No. | Ar | $R^1$ | $R^2$ | $R^3$ | $R^4$ | $R^5$ | Ratio of Z : E | Synth. method | Phys. constants (appearance) |
|---|---|---|---|---|---|---|---|---|---|
| 55 | $2,4-(CH_3)_2-\alpha-C_{10}H_5$ | $CH_3$ | H | $CH_3$ | H | $CH_3$ | 1:5 | A | m.p. ca. 172°C |
| 56 | $2,6-(CH_3)_2-\alpha-C_{10}H_5$ | $CH_3$ | H | $CH_3$ | H | $CH_3$ | Z | A | m.p. ca. 168°C |
| 57 | $2,6-(CH_3)_2-\alpha-C_{10}H_5$ | $CH_3$ | H | $CH_3$ | H | $CH_3$ | 1:3 | A | (viscous oil) |
| 58 | $6,7-(CH_3)_2-\alpha-C_{10}H_5$ | $CH_3$ | H | $CH_3$ | H | $CH_3$ | Z | A | m.p. 149—151°C |
| 59 | $6,7-(CH_3)_2-\alpha C_{10}H_5$ | $CH_3$ | H | $CH_3$ | H | $CH_3$ | E | A | m.p. ca. 199°C |
| 60 | $\beta-C_{10}H_7$ | $CH_3$ | H | $CH_3$ | H | $CH_3$ | E | A | m.p. 130—133°C |
| 61 | $\beta-C_{10}H_7$ | $CH_3$ | H | $CH_3$ | H | $CH_3$ | E | A | m.p. 129—131°C (acetate) |
| 62 | $1,4-(CH_3)_2-\beta-C_{10}H_5$ | $CH_3$ | H | $CH_3$ | H | $CH_3$ | Z | A | m.p. ca. 169°C |
| 63 | $1,4-(CH_3)_2-\beta-C_{10}H_5$ | $CH_3$ | H | $CH_3$ | H | $CH_3$ | E | A | m.p. 208—210°C |
| 64 | $5,8-(CH_3)_2-\beta-C_{10}H_5$ | $CH_3$ | H | $CH_3$ | H | $CH_3$ | E | A | m.p. 170—172°C |
| 65 | $O-CH_3{\cdot}C_6H_4$ | $CH_3$ | $CH_3$ | $CH_3$ | H | $CH_3$ | E | A | (viscous oil) |
| 66 | $2,4-(CH_3)_2{\cdot}C_6H_3$ | $CH_3$ | H | $CH_3O$ | H | $CH_3$ | E | A | m.p. 173—175°C |
| 67 | $O-CH_3{\cdot}C_6H_4$ | $CH_3$ | H | $CH_3$ | $-(CH{=}CH)_2-$ | | Z+E | A | (viscous oil) |
| 68 | $2,4-(CH_3)_2{\cdot}C_6H_3$ | $CH_3$ | H | $CH_3$ | $-(CH{=}CH)_2-$ | | Z+E | A | m.p. ca. 143°C |
| 69 | $2,5-(CH_3)_2{\cdot}C_6H_3$ | $CH_3$ | H | $CH_3$ | $-(CH{=}CH)_2-$ | | Z+E | A | (viscous oil) |
| 70 | $2-Cl-5-CH_3{\cdot}C_6H_3$ | $CH_3$ | H | $CH_3$ | $-(CH{=}CH)_2-$ | | Z+E | A | m.p. ca. 80°C |
| 71 | $O-CH_3{\cdot}C_6H_4$ | $CH_3$ | H | $CH_3$ | $CH_3$ | $CH_3$ | 3:4 | A | m.p. ca. 100°C |
| 72 | $2,5-(CH_3)_2{\cdot}C_6H_3$ | $CH_3$ | H | $CH_3$ | $CH_3$ | $CH_3$ | 1:1 | B | m.p. ca. 125°C |
| 73 | $2,5-(CH_3)_2{\cdot}C_6H_3$ | $CH_3$ | H | $CH_3$ | Et | $CH_3$ | 1:1 | B | (viscous oil) |
| 74 | $2,4-(CH_3)_2{\cdot}C_6H_3$ | $CH_3$ | H | $CH_3$ | $CH_3$ | $CH_3$ | 3:2 | B | m.p. ca. 140°C |
| 75 | $2,4-(CH_3)_2{\cdot}C_6H_3$ | $CH_3$ | H | $CH_3$ | Et | $CH_3$ | Z | B | m.p. 127—129°C |
| 76 | $2,4-(CH_3)_2{\cdot}C_6H_3$ | $CH_3$ | H | $CH_3$ | Et | $CH_3$ | 1:2 | B | (viscous oil) |
| 77 | $2,4-(CH_3)_2{\cdot}C_6H_3$ | $CH_3$ | H | $CH_3$ | n-Pr | $CH_3$ | Z | B | m.p. ca. 144°C |
| 78 | $2,4-(CH_3)_2{\cdot}C_6H_3$ | $CH_3$ | H | $CH_3$ | n-Bu | $CH_3$ | 2:1 | B | m.p. ca. 117°C |
| 79 | $2,4-(CH_3)_2{\cdot}C_6H_3$ | $CH_3$ | H | $CH_3$ | i-Bu | $CH_3$ | 1:1 | B | m.p. ca. 122°C |
| 80 | $2,4-(CH_3)_2{\cdot}C_6H_3$ | $CH_3$ | H | $CH_3$ | allyl | $CH_3$ | 1:2 | B | (viscous oil) |
| 81 | $2,4-(CH_3)_2{\cdot}C_6H_3$ | $CH_3$ | H | $CH_3$ | H | Et | E | B | m.p. 112—114°C |
| 82 | $2,4-(CH_3)_2{\cdot}C_6H_3$ | $CH_3$ | H | Et | H | Et | 5:4 | B | (viscous oil) |

| Comp. No. | Ar | $R^1$ | $R^2$ | $R^3$ | $R^4$ | $R^5$ | Ratio of Z : E | Synth. method | Phys. constants (appearance) |
|---|---|---|---|---|---|---|---|---|---|
| 83 | $2,4\text{-}(CH_3)_2\cdot C_6H_3$ | $CH_3$ | H | n-Pr | H | n-Pr | 3:2 | B | (viscous oil) |
| 84 | $2,4\text{-}(CH_3)_2\cdot C_6H_3$ | $CH_3$ | H | i-Pr | H | i-Pr | 3:5 | B | (viscous oil) |
| 85 | $2,4\text{-}(CH_3)_2\cdot C_6H_3$ | $CH_3$ | H | t-Bu | H | t-Bu | 3:2 | B | (viscous oil) |
| 86 | $2,4\text{-}(CH_3)_2\cdot C_6H_3$ | $CH_3$ | H | $CH_3$ | $-(CH_2)_3-$ | | Z+E | B | m.p. 118—120°C |
| 87 | $2,4\text{-}(CH_3)_2\cdot C_6H_3$ | $CH_3$ | H | $CH_3$ | $-(CH_2)_4-$ | | 1:2 | B | (viscous oil) |
| 88 | $2\text{-}Cl\text{-}5\text{-}CH_3\cdot C_6H_3$ | $CH_3$ | H | $CH_3$ | $CH_3$ | $CH_3$ | 1:1 | B | m.p. ca. 137°C |
| 89 | $2\text{-}Cl\text{-}5\text{-}CH_3\cdot C_6H_3$ | $CH_3$ | H | $CH_3$ | Et | $CH_3$ | E | B | m.p. 129—131°C |
| 90 | $2\text{-}Cl\text{-}5\text{-}CH_3\cdot C_6H_3$ | $CH_3$ | H | $CH_3$ | n-Pr | $CH_3$ | 1:1 | B | (viscous oil) |
| 91 | $2\text{-}Cl\text{-}5\text{-}CH_3\cdot C_6H_3$ | $CH_3$ | H | $CH_3$ | n-Bu | $CH_3$ | E | B | m.p. ca. 80°C |
| 92 | $2\text{-}Cl\text{-}5\text{-}CH_3\cdot C_6H_3$ | $CH_3$ | H | $CH_3$ | i-Bu | $CH_3$ | E | B | m.p. ca. 132°C |
| 93 | $2\text{-}Cl\text{-}5\text{-}CH_3\cdot C_6H_3$ | $CH_3$ | H | $CH_3$ | allyl | $CH_3$ | E | B | m.p. 126—128°C |
| 94 | $3\text{-}Cl\text{-}5\text{-}CH_3\cdot C_6H_3$ | $CH_3$ | H | $CH_3$ | H | Et | E | B | m.p. ca. 95°C |
| 95 | $2\text{-}Cl\text{-}5\text{-}CH_3\cdot C_6H_3$ | $CH_3$ | H | Et | H | Et | E | B | m.p. ca. 130°C |
| 96 | $2\text{-}Cl\text{-}5\text{-}CH_3\cdot C_6H_5$ | $CH_3$ | H | n-Pr | H | n-Pr | 1:1 | B | (viscous oil) |
| 97 | $2\text{-}Cl\text{-}5\text{-}CH_3\cdot C_6H_3$ | $CH_3$ | H | i-Pr | H | i-Pr | 3:5 | B | (viscous oil) |
| 98 | $2\text{-}Cl\text{-}5\text{-}CH_3\cdot C_6H_3$ | $CH_3$ | H | t-Bu | H | t-Bu | 1:1 | B | (viscous oil) |
| 99 | $2\text{-}Cl\text{-}5\text{-}CH_3\cdot C_6H_3$ | $CH_3$ | H | $CH_3$ | $-(CH_2)_3-$ | | E | B | m.p. 143—145°C |
| 100 | $2\text{-}Cl\text{-}5\text{-}CH_3\cdot C_6H_3$ | $CH_3$ | H | $CH_3$ | $-(CH_2)_4-$ | | 1:1 | B | (viscous oil) |
| 101 | $C_6H_5$ | Et | H | $CH_3$ | H | $CH_3$ | 3:1 | A | (viscous oil) |
| 102 | $O\text{-}Cl\cdot C_6H_4$ | Et | H | $CH_3$ | H | $CH_3$ | E | A | m.p. 106—108°C |
| 103 | $2,4\text{-}(CH_3)_2\cdot C_6H_3$ | Et | H | $CH_3$ | H | $CH_3$ | Z | A | m.p. 113—115°C |
| 104 | $2,4\text{-}(CH_3)_2\cdot C_6H_3$ | Et | H | $CH_3$ | H | $CH_3$ | 2:1 | A | m.p. ca. 95°C |
| 105 | $2,6\text{-}(CH_3)_2\text{-}\alpha\text{-}C_{10}H_5$ | Et | H | $CH_3$ | H | $CH_3$ | 1:1 | A | (viscous oil) |
| 106 | $C_6H_5$ | n-Pr | H | $CH_3$ | H | $CH_3$ | E | A | m.p. 98—101°C |
| 107 | $2,4\text{-}(CH_3)_2\cdot C_6H_5$ | n-Pr | H | $CH_3$ | H | $CH_3$ | 1:1 | A | (viscous oil) |
| 108 | $2,4\text{-}(CH_3)_2\cdot C_6H_3$ | i-Pr | H | $CH_3$ | H | $CH_3$ | 3:1 | A | (viscous oil) |
| 109 | $P\text{-}Cl\cdot C_6H_5$ | 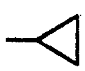 | H | $CH_3$ | H | $CH_3$ | E | A | m.p. ca. 128°C |
| 110 | $C_6H_5$ | $CF_3$ | H | $CH_3$ | H | $CH_3$ | E | A | m.p. ca. 132°C |
| 111 | $C_6H_5$ | $CO_2Et$ | H | $CH_3$ | H | $CH_3$ | E | A | m.p. 127—128°C |
| 112 | $P\text{-}Cl\cdot C_6H_4$ | $CO_2Et$ | H | $CH_3$ | H | $CH_3$ | E | A | m.p. 155—157°C |
| 113 | $2,4\text{-}Cl_2\cdot C_6H_3$ | $CO_2Et$ | H | $CH_3$ | H | $CH_3$ | Z | A | m.p. 173—176°C |

| Comp. No. | Ar | $R^1$ | $R^2$ | $R^3$ | $R^4$ | $R^5$ | Ratio of Z : E | Synth. method | Phys. constants (appearance) |
|---|---|---|---|---|---|---|---|---|---|
| 114 | 2,4-$Cl_2$·$C_6H_3$ | $CO_2Et$ | H | $CH_3$ | H | $CH_3$ | E | A | m.p. ca. 197°C |
| 115 | $C_6H_5$ | $C_6H_5$ | H | $CH_3$ | H | $CH_3$ | — | A | m.p. ca. 175°C |
| 116 | O-Cl·$C_6H_4$ | p-Cl·$C_5H_4$ | H | $CH_3$ | H | $CH_3$ | Z+E | A | m.p. 194—197°C |
| 117 | $C_6H_5$ | $C_6H_5$—$CH_2$ | H | $CH_3$ | H | $CH_3$ | Z+E | A | (viscous oil) |
| 118 | O-Et·$C_6H_4$ | $CH_3$ | H | $CH_3$ | H | $CH_3$ | 1:9 | A | m.p. 117—119°C |
| 119 | 2,4,6-$(CH_3)_3$·$C_6H_2$ | $CH_3$ | H | $CH_3$ | H | $CH_3$ | E | A | m.p. 186—189°C |
| 120 | 2,4,6-$(CH_3)_3$·$C_6H_2$ | $CH_3$ | H | $CH_3$ | H | $CH_3$ | Z | A | (viscous oil) |
| 121 | 2,4-$(CH_3)_2$·$C_6H_3$ | $CH_3$ | H | $CH_3$ | H | H | Z+E | A | (viscous oil) |
| 122 | 2,4-$(CH_3)_2$·$C_6H_3$ | $CH_3$ | H | H | H | H | E | A | m.p. ca. 125°C |
| 123 | 2,6-($Cl_2$·$C_6H_3$ | $CH_3$ | H | $CH_3$ | H | $CH_3$ | Z+E | A | m.p. ca. 135°C |
| 124 | O-$(CH_3)_2$N·$C_6H_4$ | $CH_3$ | H | $CH_3$ | H | $CH_3$ | Z+E | A | m.p. ca. 140°C |
| 125 | O-$CH_3$·$C_6H_4$ | $CH_3$ | H | $CH_3$ | H | H | 1:1 | A | m.p. ca. 76°C |
| 126 | O-Cl·$C_6H_4$ | $CH_3$ | H | $CH_3$ | $CH_3$ | $CH_3$ | Z+E | A | m.p. ca. 110°C |

Remarks: Et = —$C_2H_5$, Pr = —$C_3H_7$, Bu = —$C_4H_9$

Example 4

A wettable powder is mixed, comprising 50% of the compound (3), 2% of sodium lignin sulfonate, 3% of white carbon, 5% of polyoxyethylene alkylaryl ether and 40% of clay. It is diluted with water 1000 to 3000 times, and sprayed at an application rate of 10 to 20 *l* per are.

Example 5

A dust is mixed comprising 3% of the compound (9), 0.1% of aluminium stearate and 96.9% of clay. It is dusted at an application rate of 300 to 500 g per are.

Example 6

A granule preparation comprising 5% of the compound (18), 5% of gum arabic, 30% of bentonite and 60% of talc mixed and granuled. It is directly applied at an application rate of 300 g to 500 g per are.

Example 7

An emulsifiable concentrate, is prepared containing 20% of the compound (20), 75% of xylene and 5% of polyoxyethylene alkylaryl ether. It is diluted with water 40 to 2000 times and directly applied at an application rate of 10 *l* per are.

Example 8

A wettable powder, comprising 30% of the compound (38), 5% of sodium lignin sulfonate, 5% of polyoxyethylene alkylaryl ether and 60% of clay mixed and pulverized. It is diluted with water 40 to 2000 times and directly applied at an application rate of 10 *l* per are.

Example 9

A granule preparation, comprising a mixture composed of 10% of the compound (41), 5% of sodium lignin sulfonate and 85% of bentonite is kneaded with water and granulated. It is directly applied at an application rate of 300 to 500 g per are.

Test Example 1

An antimicrobial activity test is carried out on by means of a multiple dilution method with the use of an agar medium, in accordance with the procedure outlined below, on representative compounds of the present invention (indicated by the compound number as described in Example 3) as well as a control reference compound, the test results being tabulated in the table given below.

(1) *Assay medium*

A glucose-bouillion agar medium or potato sucrose agar medium (employed merely for the test microorganism No. 5)

(2) *Preparation of antimicrobial agents*

A 40 mg portion of the test compound is dissolved in a mixture of 0.5 ml of N,N-dimethylformamide and 9.5 ml of acetone, and diluted with sterilized water to a concentration of 1000 μg/ml (the concentration in the media is 1/10).

(3) *Test microorganisms*

1) *Pyricularia oryzae* IFO 5279, the fungus causing rice blast.
2) *Helminthosporium sigmoideum* IFO 4867, the fungus causing stem rot on rice.
3) *Helminthosporium oryzae,* the fungus causing Helmithosporium leaf spot on rice.
4) *Pellicularia sasakii* IFO 6330, the fungus causing sheath blight on rice.
5) *Phytophora capsici* IFO 8386, the fungus causing downy mildew on cucumber.
6) *Botrytis cinerea,* the pathogen of gray mould on the strawberry.
7) *Sclerotinia sclerotiorum* IFO 4876, the pathogen of Sclerotinia rot.

(4) *Control reference compound*

The compound described in Example 2 of Japanese Published (unexamined) patent application No. 12876/1978; and Chemical Abstracts, Vol. 89, 1978, page 642, 89:43476q;

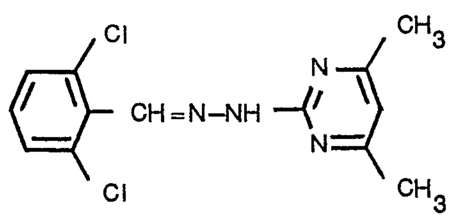

(VII)

(5) *Inoculation*

The media are inoculated with pieces of agar with hyphae, for the test microorganism Nos. 4, 5 and 6, while being inoculated by painting with bacterial fluids in the other cases.

(6) *Incubation*

Incubation is performed at 28°C for 4 days, for the test microorganism Nos. 1, 2 and 6, at 28°C for 3 days for the test microorganism Nos. 3, 4 and 7, and at 28°C for 5 to 6 days for the test microorganism No. 5.

(7) *Estimation*

The minimum inhibitory concentrations (MIC, μg/ml) are determined.

| Comp. No. | Kinds of the test microorganisms | | | | | | |
|---|---|---|---|---|---|---|---|
| | 1 | 2 | 3 | 4 | 5 | 6 | 7 |
| 2 | 3.12 | 1.56 | 25 | 50 | 3.12 | 25 | 50 |
| 3 | 0.78 | 0.39 | 6.25 | 12.5 | 3.12 | 6.25 | 1.56 |
| 7 | 3.12 | 0.78 | >100 | 100 | 1.56 | 50 | 50 |
| 8 | 0.2 | 0.2 | 12.5 | 25 | 1.56 | 6.25 | 6.25 |
| 9 | 0.39 | 0.78 | 6.25 | 12.5 | 6.25 | 12.5 | 6.25 |
| 13 | 0.78 | 0.78 | 6.25 | >100 | 12.5 | 12.5 | 6.25 |
| 15 | 1.56 | 0.78 | 50 | 100 | 6.25 | 50 | 25 |
| 16 | 0.78 | 0.78 | 100 | 50 | 12.5 | 12.5 | 25 |
| 17 | 1.56 | 6.25 | 25 | 25 | 12.5 | 25 | 12.5 |
| 18 | 1.56 | 0.78 | 25 | 12.5 | 12.5 | 6.25 | 6.25 |
| 19 | 1.56 | 1.56 | 25 | 50 | 12.5 | 25 | 12.5 |
| 20 | 0.78 | 0.78 | 25 | 12.5 | 12.5 | 6.25 | 6.25 |
| 22 | 0.78 | 0.2 | 25 | 100 | 100 | 12.5 | 6.25 |
| 23 | 0.39 | 0.2 | >100 | 25 | 50 | 12.5 | 6.25 |
| 26 | 0.78 | 0.39 | 12.5 | 12.5 | 12.5 | 12.5 | 6.25 |
| 27 | 0.39 | 0.39 | 12.5 | 50 | >100 | 25 | 6.25 |
| 28 | 0.78 | 0.39 | 50 | >100 | >100 | 25 | 6.25 |
| 31 | 0.2 | 0.1 | 25 | >100 | 25 | 50 | 3.12 |
| 34 | 1.56 | 6.25 | 12.5 | 12.5 | 25 | 6.25 | 3.12 |
| 37 | 0.2 | 0.2 | 6.25 | 12.5 | 3.12 | 6.25 | 1.56 |
| 38 | 0.39 | 0.39 | 6.25 | 12.5 | 6.25 | 6.25 | 3.12 |
| 40 | 0.39 | 0.2 | 12.5 | 25 | 6.25 | 6.25 | 3.12 |
| 41 | 0.56 | 0.78 | 25 | 100 | 25 | 12.5 | 12.5 |
| 42 | 0.78 | 0.78 | >100 | 25 | 6.25 | 50 | 3.12 |
| 43 | 0.39 | 0.2 | 50 | 6.25 | 6.25 | 6.25 | 3.12 |
| 45 | 0.78 | 0.39 | 100 | 100 | 25 | 25 | 6.25 |
| 46 | 0.78 | 0.39 | >100 | 50 | 1.25 | 12.5 | 6.25 |
| 54 | 0.05 | 0.05 | 6.25 | 25 | 6.25 | 1.56 | 1.56 |
| 57 | 0.0015 | 0.1 | >100 | >100 | 50 | 12.5 | 25 |
| 60 | 0.78 | 0.78 | 50 | 12.5 | >100 | 100 | 100 |
| 61 | 1.56 | 1.56 | 50 | 25 | >100 | 50 | 25 |

| Comp. No. | Kinds of the test microorganisms (continued) | | | | | | |
|---|---|---|---|---|---|---|---|
| | 1 | 2 | 3 | 4 | 5 | 6 | 7 |
| 67 | 0.39 | 0.78 | 3.12 | 6.25 | 6.25 | 12.5 | 0.78 |
| 68 | 0.78 | 0.78 | 25 | >100 | >100 | 25 | 3.12 |
| 69 | 0.1 | 0.1 | 12.5 | 12.5 | >100 | 12.5 | 1.56 |
| 70 | 0.025 | 0.05 | 6.25 | 25 | >100 | 12.5 | 1.56 |
| 71 | 0.2 | 0.2 | 6.25 | 12.5 | 3.12 | 12.5 | 3.12 |
| 72 | 0.78 | 25 | 12.5 | 25 | 6.25 | 12.5 | 3.12 |
| 73 | 0.78 | 25 | 12.5 | 25 | 6.25 | 12.5 | 3.12 |
| 74 | 0.78 | 0.39 | 12.5 | 25 | 3.12 | 25 | 3.12 |
| 75 | 1.56 | 1.56 | 50 | 25 | 12.5 | 6.25 | 6.25 |
| 76 | 0.39 | 0.39 | 25 | 25 | 12.5 | 12.5 | 3.12 |
| 79 | 0.39 | 0.39 | 25 | 25 | 12.5 | 12.5 | 3.12 |
| 80 | 0.39 | 0.78 | 25 | 25 | >100 | 25 | 3.12 |
| 81 | 0.39 | 0.39 | 50 | 6.25 | 25 | 25 | 1.56 |
| 82 | 0.39 | 0.39 | 25 | 25 | >100 | 25 | 3.12 |
| 87 | 100 | 0.8 | 25 | 25 | 100 | 50 | 3.12 |
| 88 | 0.39 | 0.39 | >100 | 25 | 12.5 | 12.5 | 3.12 |
| 89 | 0.025 | 0.05 | 6.25 | 6.25 | 25 | 6.25 | 1.56 |
| 93 | 0.2 | 0.2 | 6.25 | 25 | >100 | 12.5 | 3.12 |
| 94 | 0.2 | 0.2 | 6.25 | 6.25 | 6.25 | 6.25 | 3.12 |
| 95 | 0.1 | 0.1 | 12.5 | 6.25 | >100 | 6.25 | 1.56 |
| 99 | 0.78 | 0.78 | 6.25 | 12.5 | 25 | 6.25 | 3.12 |
| 100 | 100 | 0.78 | 25 | 25 | >100 | 100 | 3.12 |
| 102 | 0.78 | 0.39 | 12.5 | 50 | 25 | 12.5 | 6.25 |
| 104 | 1.56 | 0.78 | 50 | 100 | 100 | 25 | 25 |
| 108 | 1.56 | 0.78 | >100 | 50 | 25 | 25 | 1.25 |
| 118 | 0.39 | 0.39 | 12.5 | 3.12 | 12.5 | 6.25 | 1.56 |
| 119 | 0.39 | 0.39 | >100 | 50 | 25 | 25 | 12.5 |
| 125 | 6.25 | 1.56 | 25 | >100 | 100 | 12.5 | >100 |
| 126 | 0.39 | 0.39 | 6.25 | 25 | 3.12 | 12.5 | 3.12 |
| C.R* | 3.12 | 1.56 | 100 | 50 | >100 | >100 | 25 |

Remarks:
*): Control reference compound (VII)

Test Examples 2

The effect in controlling rice blast upon application to the stems and leaves of plants is investigated by the following testing method. The results obtained are tabulated in the table below, the compounds tested being indicated by the compound number as given in Example 3:

I. *Testing method*

1. Pathogen: *Pyricularia oryzae*

2. Plant to be tested: Rice, species Asahi No. 4 planted in a 9-cm pot with 10 seedlings about 32-days old.

3. Inoculation: Through natural infection from leaves affected by rice blast.

4. Treatment with antimicrobial agents: A test compound is compounded in accordance with the procedure of Example 7, diluted at the fixed concentration, supplimented with 0.2% of a spreader (Dyne, trademark of Takeda Chemical Ind.), and applied 2 days after initiation of inoculation.

5. Partition: 2 pots per section.

6. Examination: Examination is carried out in accordance with "Criteria for the Ratio of Leaf-Blast Affected Surface Area" (Pages 4—7) in "Criteria for Surveying the Incidence of Diseases and pests" published by the Japanese Associaton of Plant Protection (2nd February, 1974), 7 days after inoculation.

II. *Test results.*

| Comp. No. | Concn. ppm. | Affected surface area ratio, % | Comp. No. | Concen. ppm. | Affected surface area ratio, % |
|---|---|---|---|---|---|
| 1 | 500 | 2 | 15 | 500 | 0 |
| 2 | 500 | 0 | 16 | 500 | 0 |
| 3 | 500 | 0 | 17 | 500 | 0 |
| 4 | 500 | 2 | 18 | 500 | 0 |
| 5 | 500 | 0 | 19 | 500 | 2 |
| 6 | 500 | 0 | 20 | 500 | 0 |
| 7 | 500 | 0 | 21 | 500 | 0 |
| 8 | 500 | 1 | 22 | 500 | 1 |
| 9 | 500 | 0 | 23 | 500 | 3 |
| 10 | 500 | 1 | 24 | 500 | 3 |
| 11 | 500 | 2 | 25 | 500 | 2 |
| 12 | 500 | 0 | 26 | 500 | 0 |
| 13 | 500 | 1 | 27 | 500 | 0 |
| 14 | 500 | 2 | 28 | 500 | 0 |

*Test results* (continued)

| Comp. No. | Concn. ppm | Affected surface area ratio, % | Comp. No. | Concn. ppm | Affected surface area ratio, % |
|---|---|---|---|---|---|
| 29 | 500 | 0 | 60 | 500 | 0 |
| 30 | 500 | 1 | 61 | 500 | 2 |
| 31 | 500 | 1 | 64 | 500 | 1 |
| 33 | 500 | 1 | 67 | 500 | 0 |
| 34 | 500 | 3 | 68 | 500 | 0 |
| 35 | 500 | 0 | 69 | 500 | 0 |
| 36 | 500 | 2 | 70 | 500 | 0 |
| 37 | 500 | 0 | 71 | 500 | 0 |
| 38 | 500 | 0 | 72 | 500 | 0 |
| 39 | 500 | 0 | 73 | 500 | 1 |
| 40 | 500 | 0 | 74 | 500 | 0 |
| 41 | 500 | 0 | 75 | 500 | 0 |
| 42 | 500 | 3 | 76 | 500 | 0 |
| 43 | 500 | 1 | 77 | 500 | 0 |
| 45 | 500 | 0 | 78 | 500 | 0 |
| 46 | 500 | 0 | 79 | 500 | 3 |
| 48 | 500 | 0 | 80 | 500 | 0 |
| 49 | 500 | 0 | 81 | 500 | 1 |
| 50 | 500 | 1 | 82 | 500 | 0 |
| 51 | 500 | 3 | 83 | 500 | 2 |
| 52 | 500 | 3 | 88 | 500 | 1 |
| 53 | 500 | 0 | 89 | 500 | 0 |
| 54 | 500 | 0 | 90 | 500 | 2 |
| 55 | 500 | 0 | 91 | 500 | 0 |
| 56 | 500 | 0 | 94 | 500 | 0 |
| 57 | 500 | 0 | 95 | 500 | 0 |

*Test results* (continued)

| Comp. No. | Concn. ppm | Affected surface area ratio, % | Comp. No. | Concn. ppm | Affected surface area ratio % |
|---|---|---|---|---|---|
| 96 | 500 | 1 | 120 | 500 | 0 |
| 99 | 500 | 0 | 121 | 500 | 0 |
| 101 | 500 | 0 | 122 | 500 | 0 |
| 102 | 500 | 1 | 123 | 500 | 0 |
| 103 | 500 | 0 | 125 | 500 | 0 |
| 104 | 500 | 0 | 126 | 500 | 0 |
| 105 | 500 | 2 | CR-1* | 20 | 5 |
| 107 | 500 | 0 | CR-2** | 500 | 8 |
| 108 | 500 | 1 | N-t*** | — | 30 |
| 118 | 500 | 0 | | | |
| 119 | 500 | 0 | | | |

Remarks:
*): Blasticidin S(Bla-S, trade mark) employed as control reference.

**): EDDP (Hinosan, trade mark) employed as control reference.

***): Non-treated.

Test Example 3

The effect of controlling rice blast to be developed upon applied on the water surfaces is investigated by the following testing method, and the results obtained are tabulated in the table below, the compounds tested being indicated by the compound number given in Example 3.

1. *Testing method*

1. Pathogen: the same as described in Test Example 2.

2. Plant to be tested: The same as described in Test Example 2, except that the rice plant is grown in an 1/10000 Wagner pot.

3. Inoculation: The same as described in Test Example 2.

4. Treatment with antimicrobial agents: The test compound is compounded in accordance with the procedure of Example 7, and applied to irrigated water, either directly at the predetermined concentration or after being diluted at such concentration. Inoculation is initiated 2 days after the application.

5. Partition: The same as described in Test Example 2.

6. Examination: The same as described in Test Example 2.

II. *Test results*

| Comp. No. | Applied amount g/10a | Affected surface area ratio, % | Comp. No. | Applied amount g/10a | surface area ratio, % |
|---|---|---|---|---|---|
| 2 | 300 | 1 | 40 | 300 | 0 |
| 3 | 300 | 2 | 41 | 300 | 1 |
| 5 | 300 | 6 | 47 | 300 | 18 |
| 9 | 300 | 4 | 48 | 300 | 18 |
| 13 | 300 | 8 | 50 | 300 | 16 |
| 18 | 300 | 7 | 54 | 300 | 8 |
| 19 | 300 | 3 | 74 | 300 | 16 |
| 20 | 300 | 13 | 103 | 300 | 0 |
| 22 | 300 | 18 | 104 | 300 | 18 |
| 24 | 300 | 0 | 108 | 300 | 9 |
| 29 | 300 | 18 | CR-1* | 680 | 25 |
| 31 | 300 | 18 | CR-2** | 480 | 18 |
| | | | CR-3*** | 300 | 48 |
| | | | N-t**** | — | 80 |

Remarks:
*): IBP (Kitazin P) employed as control reference.

**): Isoprothiolane employed as control reference.

***): The compound (VII) in Test Example 1.

****): Non-treated control.

Test Examples 4

The effect of controlling Helminthosporium leaf spot on rice to be developed upon application to the stems and leaves of plants is investigated by the following testing method, the results obtained being tabulated in the table given below, in which the compounds tested are indicated by the compound number as given in Example 3:

I. Testing method

1. Pathogen: *Helminthosporium oryzae*
2. Plant to be tested: The same as described in Test Example 2.
3. Inoculation: A suspension of fungal spores (spore concentration of 1 to 2 × $10^5$/ml) formed by growing the cultures on a potato-sucrose-agar medium at 28°C for 10 days is spray-inoculated, and inoculated plants are kept for 2 days in an atmosphere at 28°C and at 100% RH (relative humidity), and are then allowed to stand in a green house.
4. Treatment with antimicrobial agents: in the same manner as described in Test Example 2, except that inoculation is effected after application and air drying.
5. Partition: The same as described in Test Example 2.
6. Examination: 7 days after inoculation, examination is carried out in accordance with the modified method of "Criteria for Judgement of the Incidence Degree of Leaf Rust, Dwarf Leaf Rust and Black Rust of Wheat" (Page 27) in "Criteria for Surveying the Incidence of Diseases and Pests" published by the Japanese Association of Plant Protection (15th January,-1974).

II. *Test results*

| Comp. No. | Concn. ppm | Incidence degree of disease, % | Comp. No. | Concn. ppm | Incidence degree of disease, % |
|---|---|---|---|---|---|
| 1 | 500 | 1 | 20 | 500 | 1 |
| 2 | 500 | 1 | 21 | 500 | 1 |
| 3 | 500 | 1 | 23 | 500 | 1 |
| 4 | 500 | 1 | 24 | 500 | 1 |
| 5 | 500 | 2 | 25 | 500 | 1 |
| 6 | 500 | 1 | 26 | 500 | 1 |
| 8 | 500 | 1 | 27 | 500 | 1 |
| 9 | 500 | 1 | 28 | 500 | 1 |
| 10 | 500 | 1 | 29 | 500 | 1 |
| 11 | 500 | 1 | 30 | 500 | 1 |
| 12 | 500 | 1 | 31 | 500 | 1 |
| 13 | 500 | 1 | 32 | 500 | 1 |
| 14 | 500 | 1 | 33 | 500 | 1 |
| 15 | 500 | 1 | 34 | 500 | 1 |
| 16 | 500 | 1 | 35 | 500 | 1 |
| 17 | 500 | 1 | 37 | 500 | 1 |
| 18 | 500 | 1 | 38 | 500 | 1 |
| 19 | 500 | 1 | 39 | 500 | 1 |

*Test results* (continued)

| Comp. No. | Concn. ppm | Incidence degree of disease, % | Comp. No. | Concn. ppm | Incidence degree of disease, % |
|---|---|---|---|---|---|
| 40 | 500 | 1 | 69 | 500 | 1 |
| 41 | 500 | 1 | 70 | 500 | 1 |
| 42 | 500 | 1 | 71 | 500 | 1 |
| 43 | 500 | 1 | 72 | 500 | 1 |
| 44 | 500 | 1 | 73 | 500 | 1 |
| 45 | 500 | 1 | 75 | 500 | 1 |
| 46 | 500 | 1 | 76 | 500 | 1 |
| 47 | 500 | 1 | 77 | 500 | 1 |
| 48 | 500 | 5 | 79 | 500 | 1 |
| 49 | 500 | 1 | 80 | 500 | 1 |
| 50 | 500 | 5 | 81 | 500 | 1 |
| 51 | 500 | 1 | 83 | 500 | 1 |
| 52 | 500 | 1 | 87 | 500 | 1 |
| 53 | 500 | 1 | 89 | 500 | 1 |
| 54 | 500 | 1 | 90 | 500 | 1 |
| 55 | 500 | 1 | 93 | 500 | 1 |
| 58 | 500 | 2 | 94 | 500 | 1 |
| 59 | 500 | 5 | 95 | 500 | 2 |
| 60 | 500 | 1 | 96 | 500 | 1 |
| 61 | 500 | 1 | 99 | 500 | 1 |
| 62 | 500 | 5 | 100 | 500 | 2 |
| 63 | 500 | 1 | 101 | 500 | 1 |
| 64 | 500 | 1 | 102 | 500 | 1 |
| 66 | 500 | 5 | 103 | 500 | 1 |
| 67 | 500 | 1 | 104 | 500 | 1 |
| 68 | 500 | 1 | 105 | 500 | 1 |

*Test results* (continued)

| Comp. No. | Concn. ppm | Incidence degree of disease, % | Comp. No. | Concn. ppm | Incidence degree of disease, % |
|---|---|---|---|---|---|
| 106 | 500 | 1 | 115 | 500 | 5 |
| 107 | 500 | 1 | 117 | 500 | 1 |
| 108 | 500 | 5 | CR-1* | 500 | 25 |
| 111 | 500 | 1 | N-t** | — | 50 |
| 112 | 500 | 5 | | | |

Remarks:
*): EDDP (Hinosan) employed as control reference
**): Non-treated control.

Test Example 5

The effect of controlling Helminthosporium leaf spot on rice to be developed upon application on water surfaces is investigated by the following testing method, the results obtained being tabulated in the table given below, in which the compounds tested are indicated by the compound number as given in Example 3:

I. *Testing method*

1. Pathogen: the same as described in Test Example 4.
2. Plant to be tested: The same as described in Test Example 2.
3. Inoculation: In the same manner as described in Test Example 4.
4. Treatment with antimicrobial agents: In the same manner as described in Test Example 4.
5. Partition: The same as described in Test Example 2.
6. Examination: In the same manner as described in Test Example 4.

II. *Test results*

| Comp. No. | Applied amount g/10a | Incidence degree of disease, % | Comp. No. | Applied amount g/10a | Incidence degree of disease, % |
|---|---|---|---|---|---|
| 2 | 300 | 0 | 39 | 300 | 5 |
| 3 | 300 | 2 | 40 | 300 | 1 |
| 9 | 300 | 1 | 41 | 300 | 2 |
| 13 | 300 | 2 | 42 | 300 | 3 |
| 16 | 300 | 1 | 43 | 300 | 3 |
| 17 | 300 | 4 | 54 | 300 | 5 |
| 18 | 300 | 5 | 74 | 300 | 2 |
| 19 | 300 | 1 | 75 | 300 | 7 |
| 20 | 300 | 1 | 103 | 300 | 1 |
| 26 | 300 | 8 | CR-1* | 680 | 68 |
| 31 | 300 | 2 | CR-2** | 500 | 68 |
| 38 | 300 | 1 | CR-3*** | 300 | 20 |
| | | | N-t**** | 300 | 80 |

Remarks:
*): IBP (Kitazin P) employed as control reference.
**): EDDP (Hinosan) employed as control reference.
***): The compound (VII) in Test Example 1.
****): Non-treated control.

Test Example 6

The effect of controlling rice sheath blight is investigated by the following testing method, the results obtained being tabulated in the table given below, in which the compounds tested are indicated by the compound number as given in Example 3.

I. *Testing method*

1. Pathogen: *Pellicularia sasakii (Rhizoctonia solani sasakii* type)

2. Plant to be test: Rice plant; species of Kinmaze, planted in 9 cm pots with 3 seedlings of 80 to 90-day old.

3. Inoculation: The peripheral portion of a mycelial colony grown on a potato-sucrose-agar medium at 28°C for 2 days is stamped out by a cork borer of 10 mm in diameter, and inserted into a stem portion of a rice plant near the ground. This is followed by maintaining the temperature at 25 to 35°C and a relative humidity of 70 to 100% after the inoculation and until the examination is effected.

4. Treatment with antimicrobial agents: In the same manner as described in Test Example 2, except that inoculation is effected after application and air-drying.

5. Partition: 2 pots per section.

6. Examination: 10 days after inoculation, the height from the base stem portion to the upper end of the diseased spot is measured to calculate a diseased spot expansion ratio in relation to that found in the non-treated section.

II. *Test results*

| Comp. No. | Concn. ppm | Diseased spot expansion rate % | Comp. No. | Concn. ppm | Diseased spot expansion rate % |
|---|---|---|---|---|---|
| 2 | 500 | 0 | 19 | 500 | 0 |
| 3 | 500 | 0 | 20 | 500 | 4 |
| 13 | 500 | 1 | 22 | 500 | 0 |
| 15 | 500 | 0 | 33 | 500 | 0 |
| 17 | 500 | 0 | 37 | 500 | 3 |
| 39 | 500 | 0 | 82 | 500 | 4 |
| 40 | 500 | 0 | 89 | 500 | 0 |
| 43 | 500 | 0 | 94 | 500 | 5 |
| 44 | 500 | 4 | 95 | 500 | 10 |
| 46 | 500 | 0 | 104 | 500 | 0 |
| 49 | 500 | 0 | CR-1* | 30 | 0 |
| 54 | 500 | 4 | CR-2** | 250 | 52 |
| 81 | 500 | 0 | N-t*** | — | 100 |

Remarks:

*): Validamycin A (Validacin) employed as control reference.

**): The compound (VII) in Test Example 1.

***): Non-treated control.

**Claims**

1. A pyrimidine derivative of the formula (I):

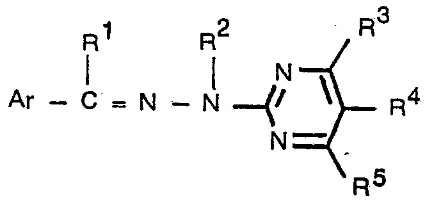

(I),

or a salt thereof, wherein Ar is phenyl or naphthyl, which may be substituted by $C_{1-4}$ alkyl, $C_{1-4}$ alkoxy, $C_{1-4}$ alkylthio, $C_{1-4}$ alkylsulfinyl, $C_{1-4}$ alkylsulfonyl, halogen, nitro, trifluoromethyl or di-$C_{1-4}$ alkylamino; $R^1$ is $C_{1-4}$ alkyl, $C_{3-5}$ cycloalkyl, trifluoromethyl, $C_{1-4}$ alkoxycarbonyl, phenyl or benzyl, and the phenyl may be substituted by halogen $R^2$ is hydrogen or $C_{1-4}$ alkyl; $R^3$, $R^4$ and $R^5$ are hydrogen, $C_{1-4}$ alkyl, $C_{2-4}$ alkenyl or $C_{1-4}$ alkoxy, or $R^3$ and $R^4$ or $R^4$ and $R^5$ combine with each other to represent trimethylene, tetramethylene or butadienylene.

2. A pyrimidine derivative as claimed in Claim 1, wherein Ar is phenyl which may be substituted by $C_{1-4}$ alkyl, $C_{1-4}$ alkoxy, $C_{1-4}$ alkylthio, halogen or trifluoromethyl; $R^1$ is $C_{1-4}$ alkyl or $C_{3-5}$ cycloalkyl; $R^3$, $R^4$ and $R^5$ are hydrogen, $C_{1-4}$ alkyl or $C_{2-4}$ alkenyl, or $R^3$ and $R^4$ or $R^4$ and $R^5$ combine with each other to represent trimethylene, tetramethylene or butadienylene.

3. A pyrimidine derivative as claimed in Claim 1, wherein Ar is phenyl substituted by $C_{1-4}$ alkyl or halogen; $R^1$ is $C_{1-4}$ alkyl; $R^2$ is hydrogen; $R^3$, $R^4$ and $R^5$ are $C_{1-4}$ alkyl or $C_{2-4}$ alkenyl, or $R^4$ and $R^5$ combine with each other to represent butadienylene.

4. A pyrimidine derivative as claimed in Claim 1, wherein Ar is phenyl substituted by $C_{1-4}$ alkyl; $R^1$ is $C_{1-4}$ alkyl; $R^2$ is hydrogen; $R^3$ and $R^5$ are $C_{1-4}$ alkyl; and $R^4$ is hydrogen.

5. A pyrimidine derivative as claimed in Claim 1 or 4 which is 4,6-dimethyl-2-[1-(2-methylphenyl)ethylidinehydrazino]-pyrimidine.

6. A pyrimidine derivative as claimed in Claim 1 or 4, which is 4,6-dimethyl-2-[1-(2,5-dimethylphenyl)ethylidenehydrazino]-pyrimidine.

7. A pyrimidine derivative as claimed in Claim 1 or 4, which is 4,6-dimethyl-2-[1-(2,4,6-trimethylphenyl)ethylidenehydrazino]pyrimidine.

8. A method of producing a pyrimidine derivative of the formula (I):

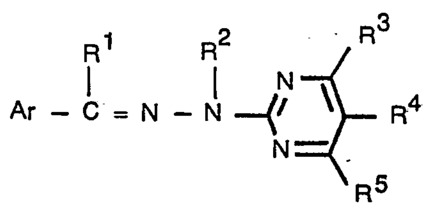

(I),

or a salt thereof, wherein Ar is phenyl or naphthyl, which may be substituted by $C_{1-4}$ alkyl, $C_{1-4}$ alkoxy, $C_{1-4}$ alkylthio, $C_{1-4}$ alkylsulfinyl, $C_{1-4}$ alkylsulfonyl, halogen, nitro, trifluoromethyl or di-$C_{1-4}$ alkylamino; $R^1$ is $C_{1-4}$ alkyl, $C_{3-5}$ cycloalkyl, trifluoromethyl, $C_{1-4}$ alkoxycarbonyl, phenyl or benzyl, and the phenyl may be substituted by halogen; $R^2$ is hydrogen or $C_{1-4}$ alkyl; $R^3$, $R^4$ and $R^5$ are hydrogen, $C_{1-4}$ alkyl, $C_{2-4}$ alkenyl or $C_{1-4}$ alkoxy, or $R^3$ and $R^4$ or $R^4$ and $R^5$ combine with each other to represent trimethylene, tetramethylene or butadienylene, which comprises reacting an aromatic ketone of the formula (II):

$$ArCOR^1 \qquad (II),$$

wherein the symbols in the formula are as defined above with 2-pyrimidylhydrazine of the formula (III):

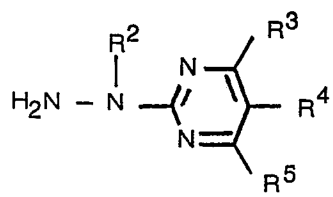

(III),

wherein the symbols in the formula are as defined above, or a salt thereof.

9. A method of producing a pyrimidine derivative of the formula (VI):

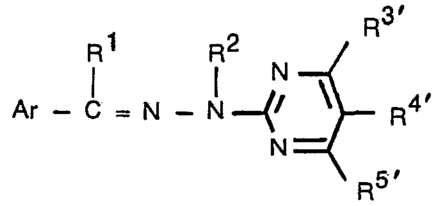

(VI),

or a salt thereof, wherein Ar is phenyl or naphthyl, which may be substituted by $C_{1-4}$ alkyl, $C_{1-4}$ alkoxy, $C_{1-4}$ alkylthio, $C_{1-4}$ alkylsulfinyl, $C_{1-4}$ alkylsulfonyl, halogen, nitro, trifluoromethyl or di-$C_{1-4}$ alkylamino; $R^1$ is $C_{1-4}$ alkyl, $C_{3-5}$ cycloalkyl, trifluoromethyl, $C_{1-4}$ alkoxycarbonyl, phenyl or benzyl, and the phenyl may be substituted by halogen; $R^2$ is hydrogen or $C_{1-4}$ alkyl, $R^{3'}$, $R^{4'}$ and $R^{5'}$ are hydrogen, $C_{1-4}$ alkyl, $C_{2-4}$ alkenyl, or $R^{4'}$ is $C_{1-4}$ alkoxy, or $R^{3'}$ and $R^{4'}$ or $R^{4'}$ and $R^{5'}$ combine with each other to represent trimethylene or tetramethylene, which comprises reacting an amidinohydrazone of the formula (IV):

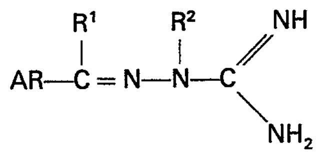

(IV),

or a salt thereof, wherein the symbols in the formula are as defined above, with a $\beta$-diketone of the formula (V):

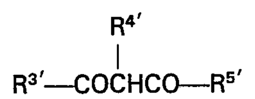

(V),

wherein the symbols in the formula are as defined above.

10. An antimicrobial agent for agricultural uses which contains as an active ingredient a pyrimidine derivative of the formula (I):

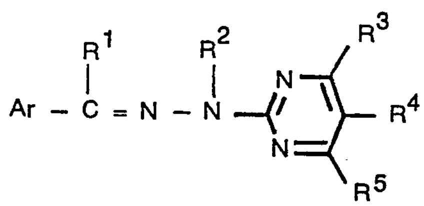

(I),

or a salt thereof, wherein Ar is phenyl or naphthyl, which may be substituted by $C_{1-4}$ alkyl, $C_{1-4}$ alkoxy, $C_{1-4}$ alkylthio, $C_{1-4}$ alkylsulfinyl, $C_{1-4}$ alkylsulfonyl, halogen, nitro, trifluoromethyl or di-$C_{1-4}$ alkylamino; $R^1$ is $C_{1-4}$ alkyl, $C_{3-5}$ cycloalkyl, trifluoromethyl, $C_{1-4}$ alkoxycarbonyl, phenyl or benzyl, and the phenyl may be substituted by halogen; $R^2$ is hydrogen or $C_{1-4}$ alkyl; $R^3$, $R^4$ and $R^5$ are hydrogen, $C_{1-4}$ alkyl, $C_{2-4}$ alkenyl or $C_{1-4}$ alkoxy, or $R^3$ and $R^4$ or $R^4$ and $R^5$ combine with each other to represent trimethylene, tetramethylene or butadienylene, together with a suitable carrier or carriers.

**Revendications**

1. Dérivés de pyrimidine ayant la formule (I)

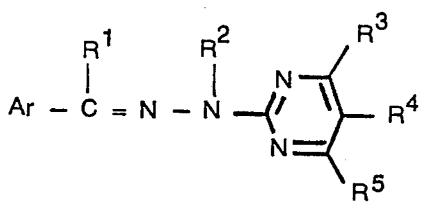

(I),

ou un des ses sels, où Ar est un groupe phényle ou naphtyle qui peut être substitué par des radicaux $C_{1-4}$ alkyle, $C_{1-4}$ alcoxy, $C_{1-4}$ alkylthio, $C_{1-4}$ alkylsulfinyle, $C_{1-4}$ alkylsulfonyle, halogéno, nitro, trifluorométhyle ou di-$C_{1-4}$ alkylamino; $R^1$ est un groupe $C_{1-4}$ alkyle, $C_{3-5}$ cycloalkyle, trifluorométhyle, $C_{1-4}$ alcoxycarbonyle, phényle ou benzyle, et le groupe phényle peut être substitué par de l'halogène; $R^2$ est de l'hydrogène ou un groupe $C_{1-4}$ alkyle; $R^3$, $R^4$ et $R^5$ sont de l'hydrogène, des groupes $C_{1-4}$ alkyle,

$C_{2-4}$ alcényle ou $C_{1-4}$ alcoxy, ou bien $R^3$ et $R^4$ ou bien $R^4$ et $R^5$, se combinent l'un avec l'autre pour représenter des groupes triméthylène, tétraméthylène ou butadiénylène.

2. Dérivé de pyrimidine selon la revendication 1, dans lequel Ar est un groupe phényle qui peut être substitué par des radicaux $C_{1-4}$ alkyle, $C_{1-4}$ alcoxy, $C_{1-4}$ alkylthio, halogéno ou trifluorométhyle; $R^1$ est un groupe $C_{1-4}$ alkyle ou $C_{3-5}$ cycloalkyle; $R^3$, $R^4$ et $R^5$ sont de l'hydrogène, des groupes $C_{1-4}$ alkyle ou $C_{2-4}$ alcényle, ou $R^3$ et $R^4$, ou $R^4$ et $R^5$, se combinent l'un avec l'autre pour représenter des groupes triméthylène, tétraméthylène ou butadiénylène.

3. Dérivé de pyrimidine selon la revendication 1, dans lequel Ar est un groupe phényle substitué par un radical $C_{1-4}$ alkyle ou halogéno; $R^1$ est un groupe $C_{1-4}$ alkyle; $R^2$ est de l'hydrogène; $R^3$, $R^4$ et $R^5$ sont des groupes $C_{1-4}$ alkyle ou $C_{2-4}$ alcényle, ou bien $R^4$ et $R^5$ se combinent l'un avec l'autre pour représenter le groupe butadiénylène.

4. Dérivé de pyrimidine selon la revendication 1, dans lequel Ar est un groupe phényle substitué par un radical $C_{1-4}$ alkyle; $R^1$ est un groupe $C_{1-4}$ alkyle; $R^2$ est de l'hydrogène; $R^3$ et $R^5$ sont des groupes $C_{1-4}$ alkyle; et $R^4$ est de l'hydrogène.

5. Dérivé de la pyrimidine selon les revendications 1 ou 4, qui est la 4,6-diméthyl-2-[1-(2-méthylphényl)éthylidène-hydrazino]-pyrimidine.

6. Dérivé de pyrimidine selon les revendications 1 ou 4, qui est la 4,6-diméthyl-2-[1-(2,5-diméthylphényl)éthylidène-hydrazino]-pyrimidine.

7. Dérivé de pyrimidine selon les revendications 1 ou 4, qui est la 4,6-diméthyl-2-[1-(2,4,6-triméthylphényl)éthylidènehydrazino)pyrimidine.

8. Procédé de préparation d'un dérivé de pyrimidine de formule (1):

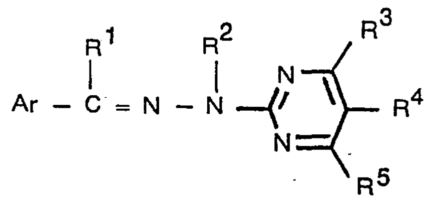

(I),

ou d'un de ses sels, où Ar est un groupe phényle ou naphtyle qui peut être substitué par des radicaux $C_{1-4}$ alkyle, $C_{1-4}$ alcoxy, $C_{1-4}$ alkylthio, $C_{1-4}$ alkylsulfinyle, $C_{1-4}$ alkylsulfonyle, halogéno, nitro, trifluorométhyle ou di-$C_{1-4}$ alkylamino; $R^1$ est un groupe $C_{1-4}$ alkyle, $C_{3-5}$ cycloalkyle, trifluorométhyle, $C_{1-4}$ alcoxycarbonyle, phényle ou benzyle, et le groupe phényle peut être substitué par de l'halogène; $R^2$ est de l'hydrogène ou un groupe $C_{1-4}$ alkyle; $R^3$, $R^4$ et $R^5$ sont de l'hydrogène, des groupes $C_{1-4}$ alkyle, $C_{2-4}$ alcényle ou $C_{1-4}$ alcoxy, ou bien $R^3$ et $R^4$, ou $R^4$ et $R^5$, se combinent l'un avec l'autre pour représenter des groupes triméthylène, tétraméthylène ou butadiénylène, qui comprend la réaction d'une cétone aromatique ayant la formule (II):

$$ArCOR^1 \qquad (II)$$

où les symboles dans la formule sont tels que définis ci-dessus, avec la 2-pyrimidylhydrazine de formule (III):

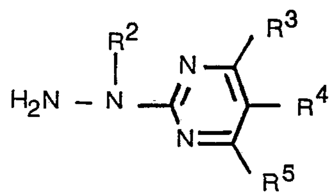

(III),

où les symboles sont tels que définis ci-dessus, ou un de ses sels.

9. Procédé de préparation d'un dérivé de pyrimidine de formule (VI)

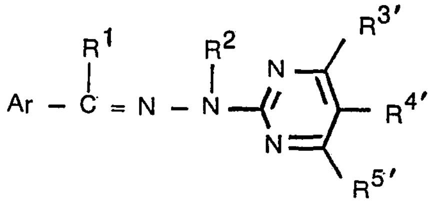

(VI),

ou d'un de ses sels, où Ar est un groupe phényle ou naphtyle qui peut être substitué par des radicaux $C_{1-4}$ alkyle, $C_{1-4}$ alcoxy, $C_{1-4}$ alkylthio, $C_{1-4}$ alkylsulfinyle, $C_{1-4}$ alkylsulfonyle, halogéno, nitro, trifluorométhyle ou di-$C_{1-4}$ alkylamino; $R^1$ est un groupe $C_{1-4}$ alkyle, $C_{3-5}$ cycloalkyle, trifluorométhyle,

$C_{1-4}$ alcoxycarbonyle, phényle ou benzyle, et le groupe phényle peut être substitué par de l'halogène; $R^2$ est de l'hydrogène ou un groupe $C_{1-4}$ alkyle; $R^{3'}$, $R^{4'}$ et $R^{5'}$ sont de l'hydrogène, des groupes $C_{1-4}$ alkyle, $C_{2-4}$ alcényle, ou bien $R^{4'}$ est un groupe $C_{1-4}$ alcoxy, ou bien $R^{3'}$ et $R^{4'}$, ou $R^{4'}$ et $R^{5'}$, se combinent l'un avec l'autre pour représenter des groupes triméthylène ou tétraméthylène, qui comprend la réaction d'une amidinohydrazone ayant la formule (IV)

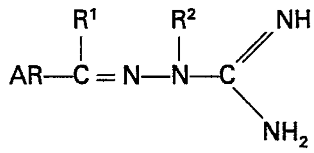

(IV),

ou un de ses sels, où les symboles dans la formule sont tels que définis ci-dessus, avec une $\beta$-dicétone ayant la formule (V)

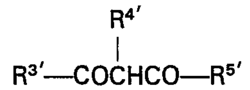

(V),

dans laquelle les symboles sont tels que défini ci-dessus.

10. Agent antimicrobien pour usages en agriculture qui contient, comme ingrédient actif, un dérivé de pyrimidine ayant la formule (I)

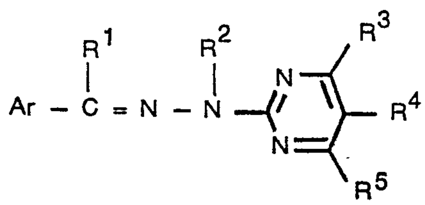

(I),

ou un de ses sels, où Ar est un groupe phényle ou naphtyle qui peut être substitué par des radicaux $C_{1-4}$ alkyle, $C_{1-4}$ alcoxy, $C_{1-4}$ alkylthio, $C_{1-4}$ alkylsulfinyle, $C_{1-4}$ alkylsulfonyle, halogéno, nitro, trifluorométhyle ou di-$C_{1-4}$ alkylamino; $R^1$ est un groupe $C_{1-4}$ alkyle, $C_{3-5}$ cycloalkyle, trifluorométhyle, $C_{1-4}$ alcoxycarbonyle, phényle ou benzyle, et le groupe phényle peut être substitué par de l'halogène; $R^2$ est de l'hydrogène ou un groupe $C_{1-4}$ alkyle; $R^3$, $R^4$ et $R^5$ sont de l'hydrogène, des groupe $C_{1-4}$ alkyle, $C_{2-4}$ alcényle, ou un groupe $C_{1-4}$ alcoxy, ou bien $R^3$ et $R^4$, ou $R^4$ et $R^5$, se combinent l'un avec l'autre pour représenter des groupes triméthylène, tétraméthylène ou butadiénylène, associé à un ou des véhicule(s) approprié(s).

**Patentansprüche**

1. Pyrimidin-Derivat der Formel (I)

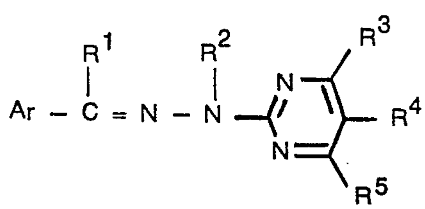

(I),

oder ein Salz desselben, worin Ar Phenyl oder Naphthyl ist, das durch $C_{1-4}$-Alkyl, $C_{1-4}$-Alkoxy, $C_{1-4}$-Alkylthio, $C_{1-4}$-Alkylsulfinyl, $C_{1-4}$-Alkylsulfonyl, Halogen, Nitro, Trifluoromethyl oder Di-$C_{1-4}$-alkylamino substituiert sein kann, $R^1$ $C_{1-4}$-Alkyl, $C_{3-5}$-Cycloalkyl, Trifluoromethyl, $C_{1-4}$-Alkoxycarbonyl, Phenyl oder Benzyl ist und das Phenyl durch Halogen substituiert sein kann, $R^2$ Wasserstoff oder $C_{1-4}$-Alkyl ist und $R^3$, $R^4$ und $R^5$ Wasserstoff, $C_{1-4}$-Alkyl, $C_{2-4}$-Alkenyl oder $C_{1-4}$-Alkoxy sind oder $R^3$ und $R^4$ oder $R^4$ und $R^5$ gemeinsam Trimethylen, Tetramethylen oder Butadienylen bezeichnen.

2. Pyrimidin-Derivat nach Anspruch 1, dadurch gekennzeichnet, daß Ar Phenyl ist, das durch $C_{1-4}$-Alkyl, $C_{1-4}$-Alkoxy, $C_{1-4}$-Alkylthio, Halogen oder Trifluoromethyl substituiert sein kann, $R^1$ $C_{1-4}$-Alkyl oder $C_{3-5}$-Cycloalkyl ist und $R^3$, $R^4$ und $R^5$ Wasserstoff, $C_{1-4}$-Alkyl oder $C_{2-4}$-Alkenyl sind oder $R^3$ und $R^4$ oder $R^4$ und $R^5$ gemeinsam Trimethylen, Tetramethylen oder Butadienylen bezeichnen.

3. Pyrimidin-Derivat nach Anspruch 1, dadurch gekennzeichnet, daß Ar Phenyl ist, das durch $C_{1-4}$-Alkyl oder Halogen substituiert ist, $R^1$ $C_{1-4}$-Alkyl ist, $R^2$ Wasserstoff ist und $R^3$, $R^4$ und $R^5$ $C_{1-4}$-Alkyl oder $C_{2-4}$-Alkenyl sind oder $R^4$ und $R^5$ gemeinsam Butadienylen bezeichnen.

4. Pyrimidin-Derivat nach Anspruch 1, dadurch gekennzeichnet, daß Ar Phenyl ist, das durch $C_{1-4}$-

Alkyl substituiert ist, $R^1$ $C_{1-4}$-Alkyl ist, $R^2$ Wasserstoff ist, $R^3$ und $R^5$ $C_{1-4}$-Alkyl sind und $R^4$ Wasserstoff ist.

5. Pyrimidin-Derivat 4,6-Dimethyl-2-[1-(2-methylphenyl)ethylidenhydrazino]pyrimidin nach Anspruch 1 oder Anspruch 4.

6. Pyrimidin-Derivat 4,6-Dimethyl-2-[1-(2,5-dimethylphenyl)ethylidenhydrazino]pyrimidin nach Anspruch 1 oder Anspruch 4.

7. Pyrimidin-Derivat 4,6-Dimethyl-2-[1-(2,4,6-trimethylphenyl)ethylidenhydrazino]pyrimidin nach Anspruch 1 oder Anspruch 4.

8. Verfahren zur Herstellung eines Pyrimidin-Derivats der Formel (I)

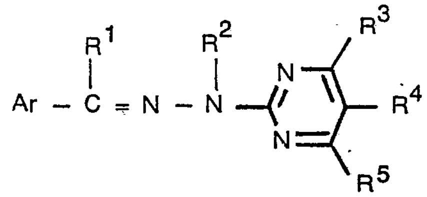

(I),

oder eines Salz desselben, worin Ar Phenyl oder Naphthyl ist, das durch $C_{1-4}$-Alkyl, $C_{1-4}$-Alkoxy, $C_{1-4}$-Alkylthio, $C_{1-4}$-Alkylsulfinyl, $C_{1-4}$-Alkylsulfonyl, Halogen, Nitro, Trifluoromethyl oder Di-$C_{1-4}$-alkylamino substituiert sein kann, $R^1$ $C_{1-4}$-Alkyl, $C_{3-5}$-Cycloalkyl, Trifluoromethyl, $C_{1-4}$-Alkoxycarbonyl, Phenyl oder Benzyl ist und das Phenyl durch Halogen substituiert sein kann, $R^2$ Wasserstoff oder $C_{1-4}$-Alkyl ist und $R^3$, $R^4$ und $R^5$ Wasserstoff, $C_{1-4}$-Alkyl, $C_{2-4}$-Alkenyl oder $C_{1-4}$-Alkoxy sind oder $R^3$ und $R^4$ oder $R^4$ und $R^5$ gemeinsam Trimethylen, Tetramethylen oder Butadienylen bezeichnen, dadurch gekennzeichnet, daß ein aromatisches Keton der Formel (II)

$$ArCOR^1 \qquad (II),$$

in der die Symble in der Formel die im Vorstehenden angegebene Bedeutung haben, mit einem 2-Pyrimidylhydrazin der Formel (III)
in der die Symbole in der Formel die im Vorstehenden angegebenen Bedeutung haben, mit einem 2-desselben umgesetzt wird.

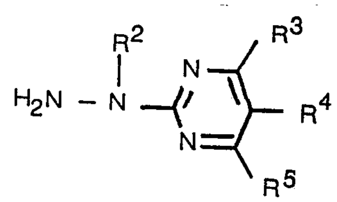

(III),

9. Verfahren zur Herstellung eines Pyrimidin-Derivats der Formel (VI)

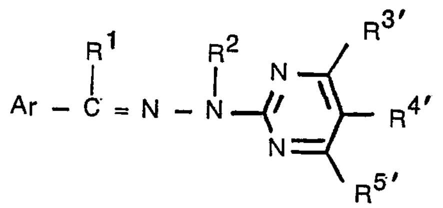

(VI),

oder eines Salz desselben, worin Ar Phenyl oder Naphthyl ist, das durch $C_{1-4}$-Alkyl, $C_{1-4}$-Alkoxy, $C_{1-4}$-Alkylthio, $C_{1-4}$-Alkylsulfinyl, $C_{1-4}$-Alkylsulfonyl, Halogen, Nitro, Trifluoromethyl oder Di-$C_{1-4}$-alkylamino substituiert sein kann, $R^1$ $C_{1-4}$-Alkyl, $C_{3-5}$-Cycloalkyl, Trifluoromethyl, $C_{1-4}$-Alkoxycarbonyl, Phenyl oder Benzyl ist und das Phenyl durch Halogen substituiert sein kann, $R^2$ Wasserstoff oder $C_{1-4}$-Alkyl ist und $R^{3'}$, $R^{4'}$ und $R^{5'}$ Wasserstoff, $C_{1-4}$-Alkyl, $C_{2-4}$-Alkenyl sind oder $R^{4'}$ $C_{1-4}$-Alkoxy ist oder $R^{3'}$ und $R^{4'}$ oder $R^{4'}$ und $R^{5'}$ gemeinsam Trimethylen oder Tetramethylen bezeichnen, dadurch gekennzeichnet, daß ein Amidinohydrazon der Formel (IV)

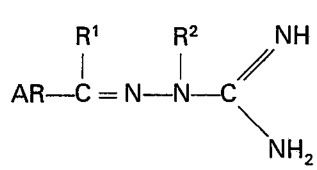

(IV)

oder ein Salz desselben, wobei die Symbole in der Formel die im Vorstehenden angegebene Bedeutung haben, mit einem $\beta$-Diketon der Formel (V)

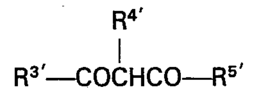

(V)

in der die Symbole in der Formel die im Vorstehenden angegebene Bedeutung haben, umgesetzt wird.

10. Antimikrobielles Mittel zur Verwendung in der Landwirtschaft, enthaltend als Wirkstoff ein Pyrimidin-Derivat der Formel (I)

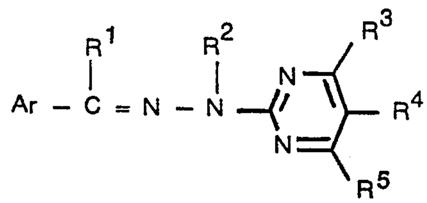

(I),

oder ein Salz desselben, worin Ar Phenyl oder Naphthyl ist, das durch $C_{1-4}$-Alkyl, $C_{1-4}$-Alkoxy, $C_{1-4}$-Alkylthio, $C_{1-4}$-Alkylsulfinyl, $C_{1-4}$-Alkylsulfonyl, Halogen, Nitro, Trifluoromethyl oder Di-$C_{1-4}$-alkylamino substituiert sein kann, $R^1$ $C_{1-4}$-Alkyl, $C_{3-5}$-Cycloalkyl, Trifluoromethyl, $C_{1-4}$-Alkoxycarbonyl, Phenyl oder Benzyl ist und das Phenyl durch Halogen substituiert sein kann, $R^2$ Wasserstoff oder $C_{1-4}$-Alkyl ist und $R^3$, $R^4$ und $R^5$ Wasserstoff, $C_{1-4}$-Alkyl, $C_{2-4}$-Alkenyl oder $C_{1-4}$-Alkoxy sind oder $R^3$ und $R^4$ oder $R^4$ und $R^5$ gemeinsam Trimethylen, Tetramethylen oder Butadienylen bezeichnen, zusammen mit einem oder mehreren geeigneten Trägern.